(19)

Europäisches
Patentamt

European
Patent Office

Office européen
des brevets

(11) **EP 3 640 633 A1**

(12) **EUROPEAN PATENT APPLICATION**

(43) Date of publication:
**22.04.2020 Bulletin 2020/17**

(51) Int Cl.:
***G01N 27/327*** (2006.01)

(21) Application number: **18200692.4**

(22) Date of filing: **16.10.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **UriSalt GmbH
6020 Innsbruck (AT)**

(72) Inventors:
• **Fuhrmann, Gerda Laura
6094 Axams (AT)**
• **Kilickiran, Pinar
6020 Innsbruck (AT)**

(74) Representative: **Engelhard, Markus
Boehmert & Boehmert
Anwaltspartnerschaft mbB
Pettenkoferstrasse 22
80336 München (DE)**

(54) **MEANS FOR THE QUANTITATIVE DETERMINATION OF CATIONIC ELECTROLYTE CONCENTRATION AND CREATININE CONCENTRATION AND OF THEIR RATIOS**

(57) The present invention relates to a single-use test strip for the quantitative determination of a concentration of a cationic electrolyte which is not sodium, and of creatinine concentration, and for the subsequent determination of their ratio, and to a non-invasive point-of-care (POC) device for detecting a disorder of electrolyte balance in patient's body. Furthermore, the present invention relates to a method for simultaneously and quantitatively determining a cationic electrolyte concentration and creatinine concentration in a patient's urine sample and to a method of detecting a disorder of electrolyte balance in a patient's body.

**EP 3 640 633 A1**

**Description**

The present invention relates to a single-use test strip for the quantitative determination of a concentration of a cationic electrolyte which is not sodium, and of creatinine concentration, and for the subsequent determination of their ratio, and to a non-invasive point-of-care (POC) device for detecting a disorder of electrolyte balance in patient's body. Furthermore, the present invention relates to a method for simultaneously and quantitatively determining a cationic electrolyte concentration and creatinine concentration in a patient's urine sample and to a method of detecting a disorder of electrolyte balance in a patient's body.

**BACKGROUND OF THE INVENTION**

The body contains a large variety of electrolytes that are essential for human and animal health. Each electrolyte serves a specific role in the body, and their well-balance is critically important for numerous physiological functions such as hydration, extra- and intracellular pH, metabolism, nerve impulses, bones density, and muscle contraction/relaxation. Important electrolytes are, beside others, cationic electrolytes, in particular positive charged ions of potassium ($K^+$), calcium ($Ca^{2+}$), magnesium ($Mg^{2+}$), copper ($Cu^{2+}$), and zinc ($Zn^{2+}$). Disorders of electrolyte balance, including depletion and/or overload of such electrolytes in the body of a patient may arise from inadequate uptake and/or disturbed excretion of these electrolytes, which may occur during various acute and chronic disease states, e.g. malfunctioning of the kidney, gastrointestinal diseases, infections that produce severe and continual diarrhea or vomiting, and due to drugs that cause loss of electrolytes in the urine (diuretics) ([1]).

Potassium (K) is the most important intracellular cation. K participates in a multitude of vital processes in every cell and K imbalance may lead to severe cellular nonresponsiveness and dangerous cardiac arrhythmia ([2]). Potassium depletion may occur due reduced K intake associated with eating disorders, as well as from increased shifts of K into cells due to e.g. insulin administration, stimulation of the sympathetic nervous system, thyreotoxicosis and familial periodic paralysis. However, the most important mechanism causing potassium depletion remains increased K loss mainly via the kidneys and/or the gastrointestinal tract ([3]). Renal potassium losses may be a consequence of acquired or genetic kidney diseases, such as Bartter's syndrome and endocrine disorders including Cushing's syndrome or caused by medications such as thiazide and osmotic diuretics, amphotericin B, penicillins and theophillin. Gastrointestinal causes occur during prolonged vomiting and/or diarrhea during infections, inflammatory, malignant and genetic bowel disease but also as a consequence of laxative abuse. Minor cases of K depletion may manifest with lesser symptoms, like muscle weakness and cramping, but severe cases can be deadly and must be treated immediately. Usually, for treatment, oral potassium supplementation is recommended; however, careful control during treatment is essential because supplemental potassium is also a common cause of K overload, for example in hospitalized patients. Potassium overload on the other hand is a frequent clinical abnormality in chronic kidney disease but often is also a consequence of high potassium intake in diets and of drugs known to raise potassium level. Further risk factors for an overload are acute kidney injury, cardiovascular disease and diabetes mellitus ([4]). K overload can be asymptomatic, meaning that it causes only vague symptoms including nausea, fatigue, muscle weakness, or tingling sensations. However, if left undiagnosed and untreated, the condition can be fatal leading to cardiac arrest and death. Patients with chronic KO are counselled to reduce dietary potassium, for which a close monitoring is necessary.

Calcium (Ca) is needed for muscle contraction, enzyme activity, and blood coagulation. Ca helps to stabilize cell membranes and is essential for the release of neurotransmitters from neurons and of hormones from endocrine glands. For bone health, sufficient bioavailable calcium is essential. Calcium depletion may be caused by Vitamin D deficiency due to insufficient intake or malassimilation as a consequence of numerous gastrointestinal diseases. Also, a variety of metabolic disorders including diabetes mellitus may be associated with an enhanced renal Ca loss ([5]). Osteoporosis is a common bone disorder that may affect females and males of any age. In particular, more than 30% of all postmenopausal women suffer from clinically relevant osteoporosis with increased risk for bone fractures ([6]). Currently, the most widely accepted method for diagnosing osteoporosis is the determination of bone mineral density (BMD). However, BMD measurements are technologically and logistically demanding, time-consuming and expensive. Other markers of osteoporosis, such as β-crosslaps and Ca excretion in 24-hour urine collections are used clinically to assess responses to treatment, however, these investigations are cumbersome and error-prone. Thus these methods are neither well suited for general population screening of increased Ca loss, nor for monitoring Ca status during osteoporosis treatment. On the other hand, a decreased excretion of Ca has been reported to be a mechanism in pre-eclampsia, which affects up to 8% of pregnancies worldwide and is one of the major causes of maternal death ([7]) demanding therefore a simpler method of monitoring Ca status.

Magnesium (Mg) is an essential mineral that participates in more than 300 enzyme reactions and also plays an important role as a second messenger. It stabilizes the resting membrane potential of muscle and nerve cells and as such plays a critical role in neuromuscular conduction, cardiac excitability and vasomotor tone ([8]).

In line with these actions are reports that magnesium depletion may be associated with increased incidence of migraine, Alzheimer's disease, coronary heart disease, hypertension, diabetes mellitus type 2. Hypomagnesiuria has also been described in up to 60% of patients with urolithiasis ([9]). A well-studied important role has been established for magnesium in pre-eclampsia and eclampsia affecting pregnant women. However, although oral magnesium sulfate administration is now an acknowledged therapeutic measure in these patient group, concerns remain about toxicity that may cause diarrhoea, vomiting, nausea, and severe irregular heartbeat to cardiac arrest ([10]). A simple method for controlling supplementation and monitoring of body Mg status is therefore of utmost importance for all affected patients.

Zinc (Zn) is an essential element in the body because it is a structural component of proteins and it is involved as a cofactor in a variety of enzymatic processes. Effects of zinc deficiency may include a diminished immune response, reduced healing and neurological disorders, as well as reduced fetal development. Zinc depletion can be caused by dietary factors, but also by inherited defects occurring in patients with gastrointestinal disorders like celiac or Crohn's disease, Wilson's disease, diabetes, chronic liver and kidney disease ([11]). Assessment of body zinc status is necessary in particular for monitoring therapies like the oral zinc therapy in Wilson's disease and for evaluating suspected nutritional inadequacy, cases of diabetes, delayed wound healing, or growth retardation.

Another essential electrolyte for many biological processes in the body is copper (Cu) which is part of several proteins. Copper depletion may be result of malnutrition and malabsorption, and due to hypoproteinemias, nephrotic syndrome, and Menkes disease. On the other hand, copper may be toxic when present in excess often causing severe liver damage ([12]). Copper overload occurs mainly in people with Wilson's disease. The main sources of copper for humans and animals are food, drinking water and copper-containing supplements. In general, copper content in the diet varies widely because foodstuffs differ greatly in their natural copper content. Factors such as season, soil quality, geography, water source and use of fertilizers influence the final copper content in food. Assessment of body copper status is necessary for monitoring therapies of Wilson's disease and for examining obstructive liver disease.

Currently, laboratory analyses are required for the assessment of electrolyte status of the body, which are logistically cumbersome prolonging time-to-result. They require medical and laboratory personnel and are neither suitable for monitoring and population screening, nor for self-testing (e.g. need for venepuncture for blood test, 24 hours urine collection). These methods do not meet the requirements of a test for a point-of care (POC) diagnostics ([13], [14]).

Electrolyte disorders can be detrimental to the patient's health and they may cause -if not treated in time-serious life-threatening problems such as renal, respiratory or circulatory failure, and even death. Timely identification of electrolyte imbalance and a simpler method of monitoring electrolyte status is therefore urgently needed.

Recently, the calculated concentration of an electrolyte (El) to creatinine (Crea) in a spot urine sample (uEl/uCrea) has emerged as a potentially promising tool for identifying and monitoring patients at risk of electrolyte disorders ([15], [16]). Creatinine is a waste product of muscle metabolism and is excreted through glomerular filtration.in urine at a relatively constant rate. Within an individual, the creatinine concentration excretion is relatively stable ([17]). Thus, for adjusting variation in volume and rate of urine production, urinary creatinine (uCrea) can possibly be taken as normalization of electrolyte concentrations

Nevertheless, the determination of uEl/uCrea, as is presently performed, is hampered by the need for sending the urine specimen to a laboratory that measures the analyte concentrations by one of the established laboratory methods. Hence, the method still requires skilled personnel, considerable organizational effort and prolonged time to result and is not suitable for point-of care (POC) diagnostics.

With the advent of electrochemical device miniaturization enabled by advanced technologies, such as screen printing or inkjet, and maturation of mobile technology, a new era of POC-testing has emerged ([13], [14]) potentially allowing the quantitative measurement of a variety of analytes as has been demonstrated for glucose in patients with diabetes mellitus ([18]).

WO96/04554A1 describes a test strip for determining calcium loss by means of determination of calcium and creatinine concentrations in urine. The method relies on the presence of two dry chemical reagent containing pads, one being sensitive for calcium and one being sensitive for creatinine, attached on the test strip. The dry chemical reagents immobilized on the pads chemically react with the analytes, if present in urine, to produce a colour change proportional to concentrations of the respective analyte(s). The readout may be done visually via a colour chart which at best is a semi-quantitative determination, or via a reflectance meter which is not amenable to a point of care setting.

The, by far, major analytical methods currently applied for determination of electrolytes concentrations in a biological sample are flame photometry, classical ion-selective potentiometry or colorimetry based on upstream chemical reactions ([19]). These methods require special instrumentation and pre-analytical sample preparation; thus, they can be performed only by trained persons. The time to result may take many hours and even days, hampering timely therapeutic decision.

Near-care diagnostic devices capable to reduce the time to result are now sometimes available in emergency units or clinical laboratories of hospitals. Examples of such devices are Radiometer ABL 800 (Radiometer, Denmark) or Cobas 6000 (Roche, CH). The systems are blood gas analysers with disposable complex cartridges containing the sensors, solutions, waste bag and even sampler. However, the analysis requires an invasive step (venipuncture) and the instruments are due to their complexity rather costly and operable only by trained persons.

The routine determination of creatinine in clinical laboratories is based on the Jaffe reaction or on the use of enzyme creatinine amidohydrolase yielding coloured compounds. It is well known that these colorimetric methods are not free from interferences and analytical problems ([20]). Highly accurate results on creatinine are provided by isotope dilution gas chromatography-mass spectrometry. This method, however, requires expensive and complex instrumentation and trained persons, thus, it cannot be considered a viable routine method.

More recently, with the aim to develop a viable routine method to determine creatinine, a few amperometric biosensors mainly relying on complex combination of three enzymes have been reported ([21]). On the market now available is the StatSensor® (Nova Biomedical, US) a portable clinical analyser that makes use of this three-enzyme system immobilized on single-use pocket sized cartridges ([22]). However, the analysis is performed with blood samples, and testing should be done by medical personnel.

Nova® Biomedical Stat Profile Critical Care Xpress (CCX) ([23]) is a benchtop point-of-care device capable of detecting creatinine with an error of approximately 11.4%, and also other analytes including K, Ca or Mg. However, the device as all others, requires blood for analysis, thus, it is invasive. In addition, it is not a portable device and is rather expensive, as it is an all-in-one system.

Considering the above information, clearly a less-invasive and less-expensive means to identify and monitor disorders of status of essential electrolytes such as K, Ca, Mg, Zn and Cu is highly desirable.

A less-invasive, less-expensive, and easy-to-use means to identify and monitor disorders of an electrolyte in a body is highly desirable.

Accordingly, it was an object of the present invention to provide for means to simultaneously determine concentrations of an electrolyte of interest and of creatinine in a patient's urine sample in a straightforward manner and to provide such means which can be easily handled. It was furthermore an object of the present invention to provide for a methodology for determining electrolyte concentration and creatinine concentration in a patient's urine sample for subsequent determining their ratio and for detecting electrolyte depletion and/or electrolyte overload that is time-efficient and can be practiced at the point-of-care (POC). It was furthermore an object of the present invention to provide for a methodology for detecting electrolyte depletion and/or electrolyte overload in a patient's body which can be practiced even by untrained persons, such as the patient him/herself. It was furthermore an object of the present invention to provide for means and a methodology for quantitatively determining electrolyte concentration and creatinine concentration in a patient's urine sample and for determining their ratio and/or to detect electrolyte depletion and/or electrolyte overload in a patient's body that is not time consuming and that can be performed as a routine operation.

In a first aspect the present invention relates to a single-use test-strip for the quantitative determination of a concentration of cationic electrolyte which is not sodium, and of creatinine concentration in a patient's urine sample, said test-strip comprising:

- a substrate which either is electrically insulating or which has an electrically insulating layer applied thereon,
- an electrode assembly applied on said substrate or on said electrically insulating layer, if present,

said electrode assembly comprising at least

- one working electrode that is selective for said cationic electrolyte;
- one creatinine-selective working electrode;
- either one joint reference electrode for both said cationic electrolyte-selective working electrode and said creatinine-selective working electrode, or a reference electrode for said cationic electrolyte-selective working electrode and a separate reference electrode for said creatinine-selective working electrode;
- optionally; one or two neutral electrodes for measuring and eliminating interferences,
- an interface for electrically connecting said electrode assembly to a read out-meter device.

In one embodiment, such determination occurs by potentiometric measurement(s).

In one embodiment, said working electrodes, said reference electrode(s) and said neutral electrode(s), if present, have been applied on said substrate or on said electrically insulating layer, if present, by a suitable deposition technique, such as printing, sputtering, evaporating, electro-less plating, affixation, gluing or lithography, preferably screen printing or ink-jet printing, thus forming an electrode assembly on said substrate or on said electrically insulating layer, and wherein said cationic electrolyte-selective working electrode comprises a cationic electrolyte-selective membrane, and said creatinine-selective working electrode comprises a creatinine-selective membrane, and wherein said neutral electrode(s) comprises(comprise) a membrane that is not selective for said cationic electrolyte and not creatinine-selective.

In one embodiment, said cationic electrolyte which is not sodium is selected from the group comprising, preferably consisting of potassium, calcium, magnesium, zinc and copper.

In one embodiment, said substrate is made of a material selected from plastic, ceramic, alumina, paper, cardboard, rubber, textile, carbon-based polymers, such as polypropylene, fluoropolymers, such as Teflon, silicon-based substrates, such as glass, quartz, silicon nitride, silicon oxide, silicon based polymers such as polydimethoxysiloxane, semiconducting materials such as elemental silicon, dielectric materials, preferably selected from organic dielectric materials such as polyimide, polycarbonate, polyvinyl chloride, polystyrene, polyethylene, polypropylene, polyester, polyethylene terephthalate, polyurethane, polyvinylidene fluoride, inorganic dielectric materials such as silicium dioxide, and wherein said electrically insulating layer, if present, is made of a dielectric material, wherein, if said electrically insulating layer is present on said substrate, said electrode assembly is located on said electrically insulating layer. More specifically, the substrate may optionally be coated with an electrically insulating layer. Such electrically insulating substrate is preferably made of a dielectric material, preferably selected from organic dielectric materials such as polyimide, polycarbonate, polyvinyl chloride, polystyrene, polyethylene, polypropylene, polyester, polyethylene terephthalate, polyurethane, polyvinylidene fluoride or inorganic dielectric materials such as silicium dioxide. In one embodiment, the electrode assembly that is applied on the substrate or on said electrically insulating substrate, if present, is part of and/or forms part of the surface of the substrate and is suitable to be brought in contact with a patient's sample, such as a urine sample. If an electrically insulating layer is present on the substrate, the electrode assembly is preferably located and applied on such electrically insulating layer.

In one embodiment, said cationic electrolyte-selective working electrode comprises a cationic electrolyte-selective membrane that comprises a cationic electrolyte-selective carrier in a polymer matrix, and said creatinine-selective membrane comprises a creatinine-selective carrier in a polymer matrix.

In one embodiment, said electrode assembly further comprises one or two neutral electrodes for measuring and eliminating interferences, wherein said neutral electrode(s) comprise a membrane comprising a polymeric matrix without any cationic electrolyte-selective carrier and without any creatinine-selective carrier.

In one embodiment, each of said electrodes in said electrode assembly has an electrical lead, respectively, wherein said electrical lead connects said electrode with said interface for electrically connecting said electrode assembly to a readout-meter device.

In one embodiment, said joint reference electrode has a surface larger than the surface of each of said working electrodes, or each of said separate reference electrodes has a surface larger than the surface of each of said working electrodes.

In a further aspect, the present invention also relates to a non-invasive point-of-care (POC) device for detecting a disorder of electrolyte balance in a patient's body, said POC device comprising:

- a readout-meter-device for the quantitative and selective measurement of cationic electrolyte concentration and creatinine concentration in a urine sample and for determining a ratio of cationic electrolyte-to-creatinine, said readout-meter-device comprising
- a receiving module for receiving an interface of a single-use test-strip according to the present invention and for establishing electrical contact between said readout-meter-device and an electrode assembly of said single-use test-strip, thus allowing the detection and transmission of electrical signal(s) from said single-use test-strip to said readout-meter-device, wherein said receiving module has electrical connectors for separately contacting each electrode via said interface of said test-strip
- a multichannel amplifier, preferably having high input resistance, for amplifying electrical signal(s) transmitted from a single-use test-strip according to the present invention
- a controller including an analog/digital converter and a storage memory, for converting electrical signals received from a single-use test-strip according to the present invention into cationic electrolyte concentration measurement(s) and creatinine concentration measurement(s) and for subsequently determining a ratio of cationic electrolyte concentration to creatinine concentration based on said cationic electrolyte concentration measurements and creatinine concentration measurements
- an output device for indicating concentration measurements and/or said ratio to a user, preferably a display, and
- a power supply.

In one embodiment, the output device may also additionally indicate other data, e. g. earlier measurements, it may also have additional functions, such as an alarm if a certain threshold of values is exceeded etc. It may comprise a save/send-function etc.

In one embodiment, said controller, for converting electrical signals received from a single-use test-strip according to the present invention into cationic electrolyte concentration measurement(s) and creatinine concentration measurement(s), makes use of pre-stored calibration information pre-stored in its storage memory. Such pre-stored calibration information is in respect of each analyte, i.e. the cationic electrolyte and creatinine. The controller additionally makes use of the Nernst equation to thereby determine concentration measurements of the respective analyte. For example, in one embodiment, where the electrode assembly comprises a first working electrode (with a first electrical lead), a joint reference electrode (with a second electrical lead), a second working electrode (with a third electrical lead) and a neutral electrode (with a fourth electrical lead), said controller uses a first electrical signal received via the first electrical connector (lead) and the second electrical connector (lead), a second electrical signal via the second electrical connector (lead) and third electrical connector (lead), and, if a neutral electrode is also present, a third electrical signal via a second electrical connector (lead) and fourth electrical connector (lead), to determine sodium and creatinine concentrations, respectively, and to subsequently calculate a ratio thereof.

In one embodiment, said receiving module is in the form of a slit, recess or well or other suitable form allowing to establish a connection to the interface of said test-strip. In one embodiment, said receiving module may be configured as an edge-connector pair or a pin-and-socket-pair.

In one embodiment, the non-invasive point-of-care (POC) device according the present invention further comprises

- a single-use test-strip according to the present invention inserted into said receiving module of said readout-meter-device by way of said interface of said single-use test-strip, thus establishing electrical contact between said electrode assembly of said test-strip and said readout-meter device.

In one embodiment, said device further comprises

- a user-interface for operating said device, and/or a memory for storing a plurality of cationic electrolyte and creatinine concentration measurements and determined ratios of cationic electrolyte concentration to creatinine concentration, and/or a connection interface, preferably a USB and/or wireless connection interface, for transferring and/or exchanging data with an external computer or external network.

In a further aspect, the present invention also relates to a method for quantitatively determining cationic electrolyte concentration and creatinine concentration in a patient's urine sample, comprising the steps:

a) providing a patient's urine sample,
b) contacting a single-use test-strip according to the present invention with said urine sample and allowing the electrode assembly of said test-strip to be wetted by and come into contact with said urine sample, optionally withdrawing the urine-wetted test-strip from said urine sample,
c) connecting said test-strip to a readout-meter-device of a point-of-care (POC) device as defined above, to assemble a point-of-care (POC) device as defined above, wherein said single-use test-strip is inserted into said receiving module of said readout-meter device, thus establishing electrical contact between said electrode assembly of said test-strip and said readout-meter-device,
wherein said connecting of said test strip to said readout-meter device of said point of care in step c) occurs either before or after step b),
d) measuring cationic electrolyte concentration and creatinine concentration in said urine sample, using said point-of-care (POC) device assembled in step c).

In a further aspect, the present invention relates to a method of detecting a disorder of electrolyte balance in a patient's body, said method comprising the steps:

- Performing the method for quantitatively determining cationic electrolyte concentration and creatinine concentration according to the present invention,
- determining a ratio of cationic electrolyte concentration to creatinine concentration using said point-of-care (POC) device,
- detecting a disorder of electrolyte balance, if said ratio of cationic electrolyte concentration to creatinine concentration in said urine sample, as determined in the previous step, is outside of, i. e. higher or lower than, a range of physiologically adequate ratio values for the respective patient.

In one embodiment, said disorder of electrolyte balance is a cationic electrolyte depletion or cationic electrolyte overload, and is, preferably, selected from:

- a potassium depletion in the plasma of a patient e.g. due to increased renal and/or gastrointestinal loss of potassium, such as may occur in acquired or genetic kidney diseases, during diuretics intake or prolonged vomiting and/or diarrhea during infections, inflammatory, malignant and genetic bowel diseases,
- a potassium overload in the plasma of a patient e.g. due to intake of drugs raising potassium level, or during acute kidney injury, cardiovascular disease and diabetes mellitus,
- a calcium depletion in the body of a patient e.g. due to metabolic disorders, Vitamin D deficiency or enhanced renal calcium loss, such as may occur in kidney stones disease, osteoporosis, and diabetes mellitus,

- a calcium overload in the body of a patient e.g. due to decreased renal calcium loss, such as may occur in pre-eclampsia during pregnancy,
- a magnesium depletion or magnesium overload in the body of a patient, such as may occur in urolithiasis, migraine, Alzheimer's disease, coronary heart disease, hypertension, diabetes mellitus type 2, pre-eclampsia and eclampsia disease,
- a zinc depletion in the body of a patient e.g. due to dietary inadequacy or gastrointestinal disorders, such as may occur in celiac or Crohn's disease, Wilson's disease, diabetes, chronic liver and kidney disease,
- a copper depletion in the body of a patient e.g. due to malabsorption and malabsorption, such as may occur during nephrotic syndrome, Menkes, disease and hypoproteinemias, and
- a copper overload in the body of a patient, due to hampered renal excretion of copper, such as may occur in Wilson's disease.

In one embodiment, said range of physiologically adequate ratio values is or has been separately determined by reference to a healthy person, preferably of the same age, sex and/or body weight.

The present inventors have provided for a simple, sensitive, non-invasive, quantitative and low-cost portable single-use test-strip for the simultaneous quantitative determination of cationic electrolyte concentration, wherein the cationic electrolyte is not sodium, and of creatinine concentration in a patient's urine sample and for a non-invasive, quantitative, low-cost and portable point-of-care (POC) device for detecting a disorder of electrolyte balance in a patient's body. The test-strip and the point-of-care device enables electrochemical measurements of urinary concentrations of cationic electrolyte and of creatinine for the subsequent calculation or determination of their respective ratio (cationic electrolyte : creatinine) to be used as a marker of a disorder of electrolyte balance.

It should be noted that the simultaneous quantitative determination of cationic electrolyte concentration and creatinine concentration occurs in an enzyme-free manner. Hence, no enzymes are used for such determination, and no products of an enzymatic reaction or other chemical reaction are measured/determined. Such determination is based, preferably solely based, on potentiometric measurements, i.e. it involves the measurement of differences in electric potential. It does not involve measurements of electric current, and it also does not involve the use of enzymes, dyes and/or dry chemical reagents that would be immobilised on pads on the test-strip. Accordingly, the test-strip(s) according to embodiments of the present invention does(do) not contain or involve the use of any enzymes or dry chemical reagents that would be immobilised on pads on the test-strip and/or react with the cationic electrolyte or with the creatinine. More preferably, the test strip(s) according to the present invention does(do) not contain dry chemical reagents for eventually reacting with a/the cationic electrolyte and creatinine, nor does(do) it(they) contain pads on which dry chemical reagents are immobilised.

The test-strip, in accordance with embodiments of the present invention is a single-use test-strip, which means that after having been used once for measurements of the cationic electrolyte concentration and of creatinine concentration, it is disposed. Hence, the test-strip in accordance with embodiments of the present invention is a disposable test-strip. It is to be used in conjunction with a non-invasive point-of-care (POC) device in accordance with the present invention for detecting a disorder of electrolyte balance in a patient's body.

A "test-strip", as used herein, in a simple embodiment, is meant to refer to a substrate that is to be brought in contact with a sample, in order for the quantitative measurement of cationic electrolyte and creatinine concentrations to be performed. The test-strip may take any form that is suitable to be contacted with a patient's sample, such as a rectangular form, square form, circular form or oval form. In one embodiment, the test-strip is planar, having a planar electrically insulating substrate on which an electrode assembly has been applied. However, in other embodiments, the test strip may also adopt other shapes and forms, such as a sheet or rod or tube, provided such form is suitable to accommodate a substrate on which an electrode array can be deposited. In one embodiment, said substrate is a planar substrate; in another embodiment, said substrate is a non-planar substrate. For example it may also be in the form of a sheet or rod or tube. In one embodiment, the electrodes of said electrode array are all arranged in a single plane on said substrate, or on said insulating layer, if present; in another embodiment, said electrodes do not necessarily have to be in a single plane, but may be arranged in different planes at an angle to each other. The only requirement is that the electrodes are arranged within the electrode array such that they can be brought in contact with urine when the test-strip is contacted with a urine sample.

In one embodiment, the test-strip according to the present invention is a single test-strip, on its own; in another embodiment, said test-strip forms part of an array of test-strips, such as may be arranged in a roll or on a disc, wherein, for each measurement, one test-strip may be used at a time. Hence, the present invention also envisages and relates to a plurality of test-strips in accordance with the present invention which are connected with each other. Hence the present invention also relates to an array of test-strips according to the present invention. In such array, each test-strip is intended for single use, but the entire array may be used for as many times as there are test-strips in such array. In one embodiment, such array of test-strips may be provided in the form of a cartridge or other dispensing device which allows the sequential use of test-strips. In one embodiment, within such array, the test-strips may be releasably connected with each other, such that, for example, if a test-strip is to be used for a measurement, it can be disconnected from the array and be used thereafter.

The term "cationic electrolyte-selective" and "creatinine-selective", as used herein, in the context of an electrode or a membrane, is meant to refer to an electrode or membrane that is specifically and selectively sensitive for the respective cationic electrolyte, e.g. $K^+$, $Ca^{2+}$, $Mg^{2+}$, $Zn^{2+}$ or $Cu^{2+}$, and/or creatinine, respectively. It should be noted that such cationic electrolyte-selective membrane or electrode is selective for the individual cationic electrolyte considered, i.e. it is capable of distinguishing between different cationic electrolytes and is selective for one of them (which has been chosen by the experimenter). Hence, a $K^+$-selective electrode or membrane is different from a $Ca^{2+}$-selective electrode or membrane. In one embodiment, such specific and selective sensitivity of an electrode is achieved by applying a cationic electrolyte-selective membrane or a creatinine-selective membrane on said electrode. In one embodiment, such cationic electrolyte-selective membrane or more generally, analyte-selective membrane is produced by applying an analyte-selective membrane solution onto the surface of the respective electrode. The application may be done by dispensing, drop casting, screen printing, spin coating or any other suitable deposition method. Such an analyte-selective membrane solution typically contains an analyte-specific carrier molecule such as an ionophor. The solution also typically contains a polymer and a solvent. Such solution may also and optionally contain other components, such as plasticizers, and/or a cation-exchanger salt. The analyte-selective solution may be prepared for example by dissolving all components in a suitable solvent. Suitable solvents are manifold, e.g. tetrahydrofuran, cyclohexanone or dimethylformamide. Once the analyte-selective membrane solution has been applied onto the surface of the electrode, the solvent is removed by drying, evaporation, etc., and what remains is a polymeric membrane which contains analyte-specific and -selective carriers.

Polymers or mixture of polymers, that may be used for the preparation of the membrane solution (from which a polymer matrix is generated) and which subsequently function as a polymer matrix in the membrane are manifold and, in one embodiment, are selected from polyvinyl chloride, polystyrene, polyacrylates, polycarbonates, polyesters, polyamides, polyurethanes, polyvinylidene chloride, polyvinyl acetate, polyvinyl alcohols, polysiloxanes, polymetacrylates, silicone elastomers, cellulose esters.

Possible are also fluorous phases of these polymers to realize extremely hydrophobic analyte-selective electrode (ASE) membranes, e.g. ion-selective electrode (ISE) membranes.

In one embodiment, the polymers are preferably of high molecular average weight to guarantee an inert character of the membrane.

In one embodiment, the percentage weight of polymeric material is from 20 to 40% in relation to the total weight of the analyte-selective membrane.

In one embodiment, one or several plasticizers are included in the membrane solution. Their role is to make the membrane softer and resistant to mechanical stress. In one embodiment, plasticizer(s) that may be used in the membrane solution, may be selected from o-nitrophenyl-octylether, bis(2-ethylhexyl)adipate, bis(2-ethylhexyl)sebacate, dioctyl sebacate, dioctylphenyl phosphonate, dimethyl phthalate, dibutyl phthalate, dioctyl phthalate, hexamethylphosphoramide, bis(1-butylpentyl)adipate, chloroparaffin. In one embodiment, the plasticizer is present in an amount sufficient to solvate analyte-selective carrier in the polymeric material. In one embodiment, the weight ratio plasticizer to analyte-selective carrier is 10:1 to 100:1. In one embodiment, the weight ratio of plasticizer:polymer in typical plasticizer:polymer mixtures is in a range of from 1:1 to 4:1. In one embodiment, the percentage weight of plasticizer is from 40 to 80% in relation to the total weight of the membrane.

Optionally, and in some embodiments, before applying the analyte-selective solution onto the surface of the respective electrode, or a polymeric material comprising KCl or a lipophilic salt, on the reference electrode, an "inner contact layer" material (also called ion-to-electron transducer) may be coated on said electrode. Without wishing to be bound by any theory, such inner contact layer has the function of avoiding the formation of capacitive layers at the electrode/membrane interface. Examples of materials suitable as "inner contact layer" are conducting polymers such as polyaniline, poly(3,4-ethylenedioxythiophene), poly(3-octylthiophene), polypyrrole, polyaniline, conductive carbon based materials such as graphene, carbon nanotubes, graphene, graphene oxide, reduced graphene oxide or metal nanoparticles. In one embodiment, they may be solubilized or dispersed in a suitable solvent and applied onto the surface of the said electrode by dispensing, drop-casting, screen printing, spin coating or any other suitable deposition method. Once the solution/dispersion has been applied onto the surface of the electrode, the solvent is removed by drying, evaporation, etc., and what remains is an "inner contact layer" on which afterwards the analyte-selective membrane is applied.

In one embodiment, the "inner contact layer" material may, optionally, also be directly included in the polymeric membrane solution of the analyte-selective membrane or polymeric material applied on the reference electrode.

In some embodiments, although the electrodes should be in electrical contact with the liquid sample, e.g. urine sample, it may be useful to prevent the electrode array and electrical leads from coming into contact with larger molecules or urinary components, such as proteins, which may interfere with and have a negative impact on the quality of the determination(s) of concentration(s). (Such worsening process, due to larger molecules is called "biofouling").

Therefore, in some embodiments, optionally, a covering membrane may applied on the portion of said test-strip which is intended to come into contact with said urine sample. An example of such a suitable covering membrane is a polycarbonate material, e.g. those sold under the Trade Mark "Nucleopore" allowing the trapping of large interfering molecules.

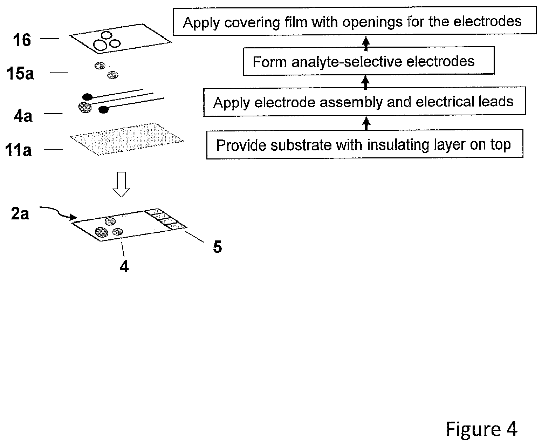

Furthermore, in some embodiments, at the end of the fabrication of a test-strip, a suitable covering film, e.g. a dielectric protective layer, such as a plastic insulating material, having openings for the electrodes can be applied on the test-strip (Figure 4) to build up a test-strip (2a) having an exposed electrode array 4 and terminal interface 5, for omitting contamination of electrical leads during storage and processing and for the commodity of use for the user.

It should be noted that, in one embodiment, the test-strip in accordance with the present invention is not a rod-like structure, but is or comprises a planar electrically insulating substrate. In one embodiment, the quantitative determination of cationic electrolyte and creatinine concentrations is not based on colorimetric or amperometric measurements. In one embodiment, it is based on potentiometric measurements. Moreover, in one embodiment, the quantitative determination of the cationic electrolyte and creatinine concentrations according to the present invention does not involve the use of enzymes, nor any oxidation/reduction reaction nor any hydrolysis of an analyte.

In embodiments according to the present invention, the single-use test-strip is to be used in connection with a point-of-care device in accordance with the present invention for detecting a disorder of electrolyte balance. For that purpose, the single-use test-strip has a suitable interface for electrically connecting the electrode assembly of said test-strip to a readout-meter-device which forms part of the point-of-care device of the present invention. Such interface may take any suitable form and may, in one embodiment, be a set of electrical contacts coming from the electrode assembly on the planar substrate. Such interface may, for example, take the form of a plug, with the electrical contacts forming part of said plug. The interface is suitable for electrically connecting the electrode assembly to a readout-meter device of a non-invasive point-of-care device for detecting a disorder of electrolyte balance, wherein such point-of-care device comprises a readout-meter-device having a receiving module for receiving an interface of a single-use test-strip according to the present invention, e.g. in the form of a recess or well or slit, for receiving the interface of the test-strip. In one embodiment, such receiving module may take the form of a socket. Typically, the receiving module is suitable to accommodate the interface of the single-use test-strip.

The term "cationic electrolyte concentration in a urine sample" or "in urine" is herein also sometimes abbreviated as "uEl". The same applies to "creatinine concentration in a urine sample" or "in urine", which is abbreviated as "uCrea". The ratio of cationic electrolyte concentration to creatinine concentration in a urine sample is herein also sometimes abbreviated as "uEl/uCrea".

The abbreviation "EMF", as used herein, refers to the electromotive force, which essentially refers to a difference in potential between two electrodes. Such electromotive force is quantitatively related via the Nernst equation to the corresponding analyte concentration in a sample. Typically, the potentiometric signal or measurement that can be taken from a test-strip according to the present invention is an electromotive force which can then be related/converted into an analyte concentration. The abbreviation "WE", as used herein, refers to a working electrode, the abbreviation "RE" refers to a reference electrode, and the abbreviation "NE" to a neutral electrode.

The term "cationic electrolyte", as used herein is meant to refer to a metal cation electrolyte which is not sodium, and which is preferably selected from the group comprising, preferably consisting of, potassium, calcium, magnesium, zinc, and copper. As used herein, the terms "potassium", "calcium", "magnesium", "zinc" and "copper" refer to the respective cationic species of such metals, i. e. $K^+$, $Ca^{2+}$, $Mg^{2+}$, $Zn^{2+}$ and $Cu^{2+}$. The term "cationic electrolyte", as used herein, is also sometimes used synonymously with "electrolyte" or "electrolyte of interest". It should, however, be understood, that such "cationic electrolyte", in accordance with the present invention is not sodium, i. e. neither in its elemental state nor as $Na^+$.

In one embodiment, said cationic electrolyte-selective working electrode comprises a cationic electrolyte-selective membrane that comprises a cationic electrolyte-selective carrier in a polymer matrix.

In one embodiment, said cationic electrolyte in "cationic electrolyte-selective" is preferably potassium, and said potassium ion-selective carriers are typically derivatives from the family of antibiotics, mono- and bis-15-crown-5 ethers. Examples of potassium-selective carriers are valinomycin, bis[(benzo-15-crown-5)-4'-ylmethyl] pimelate, and bis[(benzo-15-crown-5)-4'-ylmethyl] 2-dodecyl-2-methylmalonate, and 2-dodecyl-2-methyl-1,3-propanediyl bis[N-[5'-nitro(benzo-15-crown-5)-4'-yl]carbamate].

In one embodiment, said potassium-selective carrier molecule may be present in a percentage weight of from 0.5 % to 10%, preferably from 1% to 5%, in relation to the total weight of the potassium-selective membrane.

In one embodiment, said cationic electrolyte in "cationic electrolyte-selective" is preferably calcium, and said calcium ion-selective carriers are typically from the family of antibiotics, acyclic diamide derivatives, diazacrown ether rings, or phosphoric acid esters. Examples of calcium-selective carriers are 3,6-dioxaoctanediamide, N,N,N',N'-tetracyclohexyl-3-oxapentanediamide, bis[4-(1,1,3,3-tetramethylbutyl)phenyl)] phosphate, dodecyl phosphate, (1,7-di[2-(1-phenylazo) naphthyl]-1,4,7-trioxaheptane), and bis(octadecyl- 3-oxapentanediamide) 21-diazacrown ether.

In one embodiment, said calcium-selective carrier molecule may be present in a percentage weight of from 0.5% to 10%, preferably from 1% to 5%, in relation to the total weight of the calicum-selective membrane.

In one embodiment, said cationic electrolyte in "cationic electrolyte-selective" is preferably magnesium, and said magnesium ion-selective carriers are typically di- and triamide, β-diketone derivatives, and double-substituted diazacrown ethers. Examples of magnesium-selective carriers are N,N'-diheptyl-N,N'-dimethyl-1,4-butanediamide, N,N"-octamethylene-bis(N'-heptyl-N'-methyl-methylmalonamide, N,N"-octamethylene-bis(N'-heptyl-N'-methylmalonamide), 4,13-[bis(N-adamantylcarbamoyl)acetyl]-1,7,10,16,tetraoxa-4,13-diazacyclooctadecane, and 1,2-bis(ditolylphosphine oxide)benzene).

In one embodiment, said magnesium-selective carrier molecule may be present in a percentage weight of from 0.5% to 10%, preferably from 1% to 5%, in relation to the total weight of the magnesium-selective membrane.

In another embodiment, said cationic electrolyte in "cationic electrolyte-selective" is preferably copper, and said copper ion-selective carriers are typically derivatives from the family of macrocyclic tetrathioethers, noncyclic dithiocarbamates and hydroxamates. Examples of copper ion-selective carriers are N-(m-nitrocinnamoyl),N-(p-chlorophenyl)hydroxylamine, N,N,N',N'-tetracyclohexyl-2,2'-thiodiacetamide, o-xylylene-bis(N,N-diisobutyldithiocarbamate), 2-(1,4,8,11-tetrathiacyclotetradec-6-yloxy)hexanoic acid, and tetraethylthiuram disulfide.

In one embodiment, said copper ion-selective carrier molecule may be present in a percentage weight of from 0.5% to 10%, preferably from 1% to 5%, in relation to the total weight of the electrolyte-selective membrane.

In another embodiment, said cationic electrolyte in "cationic electrolyte-selective" is preferably zinc, and said zinc ion-selective carriers are typically derivatives from the family of alkylated thiuram disulfides, azaglutaric acid diamides. Examples of zinc-selective carriers are tetrabutylthiuram disulfide, N-benzyliminodiacetic acid bis(N-ethyl-N-cyclohexylamide) and potassium hydrotris(N-t-butyl-2-thioimidazolyl)borate.

In one embodiment, said zinc-selective carrier molecule may be present in a percentage weight of from 1% to 10%, preferably from 2% to 6%, in relation to the total weight of the electrolyte-selective membrane.

In one embodiment, prior to the potentiometric measurement, the creatinine has to be protonated to form a creatininium ion by adjusting the pH of said sample by addition of a suitable buffer. The term "creatinine-selective" is meant to encompass a selectivity for creatinine, irrespective and independent of the protonation state of said creatinine. Hence, a creatinine-selective electrode is selective for either creatinine in non-protonated form or in protonated form or both. In one embodiment, a suitable pH is below 5, and suitable buffers are manifold, e.g. they may be acetate, citrate, or phosphate. Typically, and in one embodiment, protonated creatinine-selective carriers (creatininium-selective carriers) may be selected from the family of crown ethers, α-, β-cyclodextrines, calixpyrroles, amino-pyridone and amino-pyrimidones. Examples of creatinine-selective carriers are dibenzo-30-crown-10; tri-o-octyl-β-cyclodextrin; 2,6-di-o-dodecyl-β-cyclodextrin; 1-(5,7,7-trimethyl-2-(1,3,3-trimethylbutyl)-octyl)isocytosine.

In one embodiment, the creatinine-selective carrier may be a crystalline ion-pair complex, such as creatinine tungstophosphate, creatinine molybdophosphate or creatinine picrolonate. In one embodiment, said creatinine ion-selective carrier molecule may be present in a percentage weight of from 0.5% to 10%, preferably from 1% to 5%, in relation to the total weight of the creatinine-selective membrane.

In a further aspect, the present invention relates to a non-invasive point-of-care (POC) device for detecting a disorder of electrolyte balance in a patient's body, said POC device comprising:

a readout-meter device for the quantitative and selective measurement of a cationic electrolyte concentration and creatinine concentration in a urine sample and for determining a ratio of cationic electrolyte-to-creatinine, said readout-meter device comprising

- a multichannel amplifier, preferably having high input resistance for amplifying electrical signal(s) transmitted from a single-use test-strip
- a controller including an analog/digital converter and a storage memory, for converting electrical signals received from a single-use test-strip into analyte concentration measurement(s)
- a measurement mechanism to start and, preferably, conduct a measurement of an analyte concentration
- an output device for indicating concentration measurements to a user, preferably a display
- a receiving module for receiving an interface of a single-use test-strip or of a test-strip holder unit,
- optionally, a communication unit, and
- a power supply; and

optionally a test-strip holder unit for receiving an interface of a single-use test-strip and having an interface for connecting to said receiving module and for establishing electrical contact between said readout-meter device and an electrode assembly of said single-use test-strip, thus allowing the detection and transmission of electrical signal(s) from said single-use test-strip to said readout-meter device, wherein said test-strip holder unit has electrical connectors for separately contacting each electrode via said interface of said test-strip.

In one embodiment, said POC device, preferably said readout-meter-device, further comprises a release mechanism to release said test-strip from said receiving module of the readout-meter-device or from said test-strip holder unit, if present. In one embodiment said POC-device mandatorily comprises a test-strip holder unit. In one embodiment, said measurement mechanism is selected from a measure button, such as a push button, a press button, a touch button, or a digital control button, a lever, a second pressure point of a button of the POC device or pressing a button of the POC device for a second time, and a voice-control means to initiate the measurement.

In one embodiment, said POC device further comprises a release mechanism to release said test-strip from said receiving module of the readout-meter device or from said test-strip holder unit, if present. In one embodiment, said release mechanism is selected from a button, such as a push button, a press button, a touch button, or a digital control button, a lever, a second pressure point of a button of the POC device or pressing a button of the POC device for a second time, and a voice-controlled release mechanism.

In one embodiment, said release mechanism is part of the readout-meter device, or said release mechanism is part of the test-strip holder unit. In one embodiment, said device further comprises a user-interface for operating said device, and/or a memory for storing a plurality of electrolyte concentration measurements, and/or a communication unit, preferably a USB and/or wireless communication unit, for transferring and/or exchanging data with an external computer or external network. In one embodiment, said device comprises both a port for USB connection and wireless connection. In one embodiment, said POC device comprises a test-strip holder unit comprising a main body and at least two terminal interfaces, one for receiving an interface of said test-strip and the other for contacting said receiving module of said readout-meter device, and electrical leads for electrically connecting said digital readout-meter device with said single-use test strip.

In one embodiment, said device further comprises a single-use test-strip inserted into said receiving module of said readout-meter device or into said test-strip holder unit by way of said interface of said single-use test-strip, thus establishing electrical contact between said electrode assembly of said test-strip and said readout-meter device, wherein preferably, said single-use test-strip is the single-use test-strip for the quantitative determination of a concentration of a cationic electrolyte which is not sodium and of creatinine concentration, as defined further above and below herein.

In one embodiment, said measurement is a potentiometric measurement.

In one embodiment, the quantitative determination of a concentration of a cationic electrolyte and of creatinine concentration occurs by potentiometric measurement(s).

It should be noted that, in accordance with embodiments of the present invention, the determination of the respective concentration of a cationic electrolyte which is not sodium, and of creatinine concentration occurs "in combination". By the term "in combination", it is meant that both analytes' concentrations are determined together in or from one sample, i. e. urine sample. However, this does not imply any particular order of determination: For example, such determination may occur simultaneously, in a temporarily overlapping manner or sequentially. In one embodiment, the analytes' concentrations are determined concomitantly, which essentially means that they are determined together without any substantial temporal interval between these determinations.

In one embodiment, the test-strip according to embodiments of the present invention, is a separate test-strip; in another embodiment, said test-strip forms part of an array of test-strips, such as may be arranged in a role or on disc, wherein, for each measurement, one test-strip may be used at a time.

In one embodiment, said substrate is a planar substrate; in another embodiment, said substrate is a non-planar substrate. For example, it may also be in the form of a sheet or rod or tube. In one embodiment, the electrodes of said electrode array are all arranged in a single plane; in another embodiment, said electrodes do not necessarily have to be in a single plane, but may be arranged in different planes at an angle to each other. The only requirement is that the electrodes are arranged within the electrode array such that they can be brought into contact with urine when the test-strip is contacted with a urine sample.

In one embodiment, said working electrodes, said reference electrode(s) and said neutral electrode(s), if present, have been applied on said substrate or on said electrically insulating layer, if present, by a suitable deposition technique, as defined herein, thus forming electrode assembly on said substrate or on said electrically insulating layer. The purpose of the neutral electrode(s) is to measure and eliminate interference(s). Depending on the type and nature of the(se) interference(s), however, neutral electrode(s) may be selective for such interference/interfering analyte. In one embodiment, as an example, the neutral electrode(s) is (are) proton-selective and comprise(s) a membrane(s) which is (are) proton-selective. Hence, whilst the neutral electrode(s) is (are) not cationic electrolyte-selective and not creatinine-selective, they may nevertheless be selective for other entities, e. g. protons, in particular for these other entities that give rise to interferences.

In one embodiment, the working electrode(s) may be made of any conductive material such as carbon, gold, palladium, silver, platinum, titanium, chromium, iridium, tin, their oxides or derivatives and combinations thereof, such as fluorine doped tin oxide (FTO) or indium tin oxide (ITO). In one embodiment, said electrode(s) has (have) been applied on said substrate or on said electrically insulating layer if present, by a suitable deposition technique, such as printing, sputtering, evaporating, electro-less plating, affixation, gluing or lithography, preferably screen printing or ink-jet printing.

The electrodes may be individually deposited or together.

In one embodiment, the reference electrode(s) preferably is a Ag/AgCl system, with other suitable reference material(s) providing a controlled potential and biological fluids being also possible and envisaged.

In one embodiment, said reference electrode(s) is made from the same material as the working electrodes and has a surface, which surface is coated with a stabilizing layer such as a conductive polymer e.g. polyaniline, poly(3,4-ethylenedioxythiophene), poly(3-octylthiophene), polypyrrole, or a polymeric material, e.g. polyurethane, polyvinylbutyral, polyvinylchloride, comprising Ag/AgCl/Cl$^-$ system for maintaining constant potential of the electrode.

In one embodiment, for a higher potentiometric stability, the reference electrode may be in addition coated with a stabilizing layer of polymeric material including lipophilic salts, such as anion and cation exchange materials (e. g. different tetraalkylammonium(s) and tetraphenylborates). These components of high lipophilicity exclude or minimize ion exchange with the sample solution and confer constant potential of reference electrode.

In one embodiment, said electrode assembly further comprises one or two neutral electrodes (NE) for measuring and eliminating interferences, as outlined already above. Preferably, such neutral electrode(s) comprise a membrane comprising a polymeric matrix without any cationic electrolyte-selective carrier and without any creatinine-selective carrier. Typically, said neutral electrode(s) comprise the same or similar polymer matrix as in said cationic electrolyte-selective membrane and/or creatinine-selective membrane of said working electrodes (WE) but without comprising a cationic electrolyte-selective and creatinine-selective carrier(s). The advantage associated with such neutral electrodes is, that a system incorporating such neutral electrodes is capable of additionally measuring interferences in the sample arising from other analytes, such as e. g. uric acid or ascorbic acid. Therefore, whilst the neutral electrodes are not selective for a cationic electrolyte, as defined herein, and not for creatinine, it should, however, be noted that neutral electrode(s) may be selective for analytes other than the cationic electrolyte, as defined herein, or creatinine, e. g. those analytes or entities that give rise to possible interference(s). In one embodiment, the neutral electrode(s) may be selective for protons. In another embodiment, the neutral electrode(s) may be selective for uric acid and/or ascorbic acid. Also these neutral electrodes each have an electrical lead, respectively, wherein said electrical lead connects that respective electrode with said interface for electrically connecting said electrode assembly to a readout-meter device.

In one embodiment, each of said electrodes in said electrode assembly has an electrical lead, respectively, wherein said electrical lead connects said electrode with said interface for electrically connecting said electrode assembly to a readout-meter device. In one embodiment, said electrically leads may be made of any suitable conductive material, such as carbon, gold, palladium, silver, platinum, titanium, chromium, iridium, tin, their oxides or derivatives and combinations thereof, such as FTO, ITO. In one embodiment, said electrical leads have been applied on said substrate or on said electrically insulating layer, if present by a suitable deposition technique, such as printing, sputtering, evaporating, electroless plating, affixation, gluing or lithography, preferably screen printing or ink-jet printing. The electrical leads may be individually deposited or together.

It should be noted that both the form and the positional arrangements or the electrodes on the substrate or on the insulating layer, if present, (e. g. round, oval, square, rectangular) are not critical for achieving useable results from the test-strip. Exemplary possible arrangements may for example be: WE1-WE2-RE, WE1-RE-WE2 or e. g. if a neutral electrode is present, WE1-NE-WE2-RE, WE1-WE2-NE-RE, NE-WE1-WE2-RE etc. (WE = working electrode; RE = reference electrode; NE = neutral electrode).

In one embodiment, said joint reference electrode has a surface larger than the surface of each of said working electrodes, or each of said separate reference electrodes have a surface larger than the surface of each of said working electrodes.

In one embodiment, the readout meter comprises a release mechanism to release the test-strip.

In one embodiment, the readout meter comprises a test strip holder, optionally detachable, for receiving an interface of a test-strip.

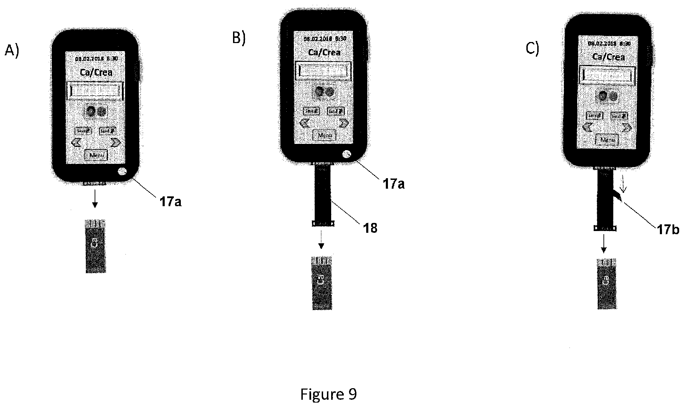

In one embodiment, the readout meter comprises a release mechanism to release said test-strip from the readout-meter device or from said test-strip holder (Figure 9, B).

In one embodiment, the readout meter comprises a test strip holder for receiving an interface of a test-strip and has a release mechanism (Figure 9, C).

In one embodiment, said release mechanism is selected from a button, such as a push button, a press button, a touch button, or a digital control button, a lever, a second pressure point of a button of the POC device or pressing a button of the POC device for a second time, and a voice-controlled release mechanism.

In one embodiment, said release mechanism is part of the readout-meter device, or said release mechanism is part of the test-strip holder unit.

In one embodiment, said release mechanism is activated after measurement for removing said test-strip from said readout-meter device or from said test-strip holder unit to avoid contact of the user with a urine sample.

In one embodiment, said device further comprises a user-interface for operating said device, and/or a memory for storing a plurality of analyte concentration measurements, and/or a communication unit, preferably a USB and/or wireless communication unit, for transferring and/or exchanging data with an external computer or external network.

In one embodiment, said device comprises both a port for USB connection and wireless connection.

In embodiments of a method of detecting a disorder of electrolyte balance in a patient's body, a disorder of electrolyte balance is detected if a ratio of cationic electrolyte concentration to creatinine concentration in a urine sample is outside of, i. e. higher or lower than, a range of physiological adequate ratio values for the respective patient. The term "a range of physiologically adequate ratio values for the respective patient", as used herein relates to a range of ratio values, i. e. ratios of cationic electrolyte concentration to creatinine concentration, which is a ratio that may be measured in humans or animals that do not have a disorder of electrolyte balance. In one embodiment, such range of physiologically adequate ratio values is or has been separately determined by reference to a healthy person, preferably of the same age, sex, nutrition, and/or body weight. It is clear to a person skilled in the art that such range of a healthy patient may be relatively broad. Exemplary, but not limiting values for such physiologically adequate ratio value ranges are for healthy adult human patients: Potassium: 5-12; Calcium: 0.05-0.2; Magnesium: > 0.25; Zinc: $0.15 \times 10^{-3}$ - $1.6 \times 10^{-3}$ Copper: $1.2 \times 10^{-5}$ - $12.8 \times 10^{-5}$

The term "non-invasive point-of-care (POC) device", as used herein, relates to a device for quantitative potentiometric determination of a concentration of a cationic electrolyte which is not sodium, and of creatinine concentration in a patient's urine sample. A POC device of the present invention comprises a readout-meter device as an analyzer unit for the quantitative and selective potentiometric measurement of said concentrations in a urine sample. In accordance with embodiments of the present invention, a POC device optionally comprises a test-strip holder unit which connects a test-strip to said readout-meter device of the POC device. The POC device is combined with a single-use test-strip as sensing unit for the quantitative determination of an analyte in urine, optionally by means of said test-strip holder unit which is interposed between said readout-meter device of said POC device and said test-strip. In one embodiment, a POC device of the present invention further comprises a measurement mechanism and/or a release mechanism. In one embodiment, said measurement mechanism and/or said release mechanism are part of the readout-meter device. In another embodiment, said measurement mechanism and/or release mechanism are part of said test-strip holder unit.

The term "readout-meter device", as used herein, relates to a device suitable and configured to quantitatively measure electrical signals received from a sample-exposed single-use test-strip connected to the readout-meter device via a receiving module of said readout-meter device, wherein optionally a test-strip holder unit is interposed between said receiving module of said readout-meter device and said test-strip. The readout-meter device according to the present invention is an electrometric device which is either a direct reading circuit or a null-balance potentiometric circuit. In many of the embodiments of the present invention, said readout-meter device is a multi-use digital potentiometric readout-meter for quantitatively determining the concentration of a cationic electrolyte and the concentration of creatinine in a patient's urine sample. The readout-meter device is part of the POC device and is configured to calculate the concentrations of said cationic electrolyte and of said creatinine in the sample based on the electrical signals and calibration information, and is also configured to output, e.g. display the results to a user. In one embodiment, such readout-meter device comprises a measuring unit such as an analog-to-digital converter to transform electrical signals received from said test-strip in digital values and an analyzing unit, such as a controller that is configured to determine potential differences in the electrode assembly from digital values , and to calculate the concentrations of said cationic electrolyte and of said creatinine based on such electrical signal(s). In one embodiment, said calibration information is initially determined for a set of test-strips and saved in an analyzing unit such as a software to perform calculations of analyte concentrations later on. In one embodiment, said measuring unit is preferably triggered by a measurement mechanism. In some embodiments, for exerting control over a measurement, the measurement mechanism may be configured for this purpose or the measuring unit or both. Furthermore, the readout-meter device comprises an output unit connected to the controller for outputting results for inspection by a user. In one embodiment, such readout-meter device further comprises a communication unit, for example a USB and/or wireless port for exchanging data with an external computer or network. The readout-meter device has an interface part for receiving the test-strip or the test-strip holder unit. In one embodiment, the readout-meter device comprises a measurement mechanism and a release mechanism.

The term "receiving module", as used herein, relates to a receiving module of a readout-meter device for receiving an interface of a test-strip or a test-strip holder unit and for establishing electrical contact between said readout-meter-device and an electrode assembly of said test-strip, thus allowing the detection and transmission of electrical signal(s) from said test-strip to said readout-meter-device, optionally via a test-strip holder unit, wherein said receiving module has electrical connectors for separately contacting each electrode via said interface of said test-strip or said test-strip holder unit. In one embodiment, said receiving module is in the form of a slit, recess or well or other suitable form allowing to establish a connection to the interface of said test-strip or said test-strip holder unit. In one embodiment, said receiving module may be configured as an edge-connector pair or a pin-and-socket-pair. In one embodiment, a single-use test-strip according to the present invention is inserted into said receiving module of said readout-meter device by way of said interface of said single-use test-strip, thus establishing electrical contact between said electrode assembly of said test-strip and said readout-meter device. In one embodiment, a single-use test-strip according to the present invention is inserted into said test-strip holder unit which is connected to said receiving module of said readout-meter device by way of said interface of said single-use test-strip, thus establishing electrical contact between said electrode assembly of said test-strip and said readout-meter device via said test-strip holder unit. In one embodiment, said readout-meter device has a receiving module which is capable of receiving an interface of a single-use test-strip. In one embodiment, said readout-meter device has a receiving module which is capable of receiving an interface of a test-strip holder unit. In one embodiment, said readout-meter device has a receiving module which is capable of receiving an interface of a single-use test-strip, and has a receiving module which is capable of receiving an interface of a test-strip holder unit. In one embodiment, said readout-meter device has a receiving module which is capable of receiving an interface of a single-use test strip and/or a test-strip holder unit.

The term "interface", as used herein, relates to a first interface which is part of a receiving module of a readout-meter device, wherein said first interface is capable of establishing a contact to a second interface which is part of an optional test-strip holder unit capable of connecting to said first interface of said receiving module of said readout-meter device, and wherein said first interface is capable of establishing a contact to an interface of a test-strip referred to as a fourth interface. The term also relates to a third interface which is part of the test-strip holder unit capable of connecting to a fourth interface which is part of a test-strip. Said fourth interface which is part of said test-strip may be connected to said third interface of said test-strip holder unit or may also be directly connected to said first interface which is part of said receiving module of said readout-meter device.

The term "communication unit", as used herein, relates to an optional unit of the point-of-care (POC) device for transmitting results wired and/or wireless to a reading unit or network, for example a software app developed for data management and for easy communication between user and doctor, or a server connected to a hospital information system (HIS) or laboratory information system (LIS). In one embodiment, such communication unit comprises an USB port and/or a bluetooth module.

The term "measurement mechanism", as used herein, relates to a feature of the POC device that is used to initiate and, preferably, conduct the measurement of the concentration of a cationic electrolyte and of the concentration of creatinine in a patient's urine sample. An exemplary measurement mechanism is a measure button, such as a push button, a press button, a touch button, or a digital control button, a lever, a second pressure point of a different button of the POC device or pressing a button of the POC device for a second time, or a voice-controlled means to initiate the measurement. The term "measurement mechanism", as used herein, may also relate to an automatic initiation of the measurement at a defined point of time, after the sensing unit senses a sample. In one embodiment, said measurement mechanism preferably operates in conjunction with a measuring unit to conduct a measurement.

The term "time-controlled manner", as used herein, relates to a mechanism of performing a measurement, wherein a measurement, after having been started, preferably at a defined point in time, is subsequently performed over a defined duration, wherein said defined duration is a measurement period. In one embodiment, the mean value is calculated for values acquired during a measurement period. In one embodiment, said mean value is displayed by means of the POC device. In one embodiment, said mean value is used for subsequent calculations of the concentration of a cationic electrolyte and of the concentration of creatinine and the ratio thereof, and/or body status of said cationic electrolyte and of said creatinine.

The term "measure button", as used herein, relates to an exemplary measurement mechanism, which is a button at the POC device that initiates a measurement when pushed (push button), pressed (press button), or touched (touch button). The term also comprises a digital control button such as a displayed button icon on a touchscreen.

The term "to initiate a measurement", when used in conjunction with a measurement mechanism, refers to an action whereby a measurement is started at a defined point in time, namely when the measurement mechanism is actuated.

The term "to conduct a measurement", when used in conjunction with a measurement mechanism, refers to an action whereby a measurement, after having been started at a defined point in time, is subsequently performed until its completion, and such performing includes control over various measurement parameters, such as the duration of measurement period or values' variance of the received electrical signals in a defined measurement period. In one embodiment, a measurement is conducted in a time-controlled manner. In one embodiment, a measurement is preferably conducted by a measurement mechanism in conjunction with a measuring unit, as defined further above. In some embodiments, for exerting control over a measurement, the measurement mechanism may be configured for this purpose or the measuring unit or both.

The term "release mechanism", as used herein, relates to a means or a mechanism that allows to release a test-strip from a receiving module of a readout-meter device or from a test-strip holder unit. An exemplary release mechanism is a button, such as a push button, a press button, a touch button, or a digital control button, a lever, a second pressure point of a button of the POC device or pressing a button of the POC device for a second time, e.g. a measure button, or a voice-controlled release mechanism. A release mechanism may be part of a readout-meter device or of a test-strip holder unit. The term "release mechanism", as used herein, may also relate to an automatic release of the test-strip, when the measurement is finished.

The term "second pressure point of a button", as used herein, relates to a feature of a button which has a certain function to evoke also a second, different function, wherein said second, different function can be initiated by pressing said button at a second pressure point of said button. For example, a button may be pushed to a defined first level to evoke a first function of said button, and may be pushed to a defined second level to evoke a second function of said button, wherein said first and said second level are different. For example, a second pressure point of a button may be activated by pressing, pushing, or touching said button at said second pressure point. The term may also relate to a second position of a lever which allows to activate a second function of said lever. The term also comprises a second function of a button, wherein said button can be activated to perform a different function depending on the length of time that said button is activated, for example, by pushing, pressing or touching.

The term "pressing a button for a second time", as used herein, relates to a feature of a button which has a certain first function to evoke also a second, different function, wherein said second, different function can be initiated by pressing said button for a second time. The term also comprises a feature of a button, wherein a button can be triggered to evoke different functions depending on the length of time said button is triggered by means of pressing, pushing, touching, or the like.

The term "voice-controlled", as used herein, relates to a mechanism that allows to control the POC device by voice. Voice-control is an optional feature of the POC device enabled by an optional voice-control unit. In one embodiment, said optional voice-control unit can be used to start a measurement, i.e. as measurement mechanism, and/or to release a test-strip from the test-strip holder unit, i.e. as release mechanism.

The term "test-strip holder unit", as used herein, refers to a unit which is optionally a part of a POC device according to embodiments of the present invention, and electrically connects a digital readout-meter device with a single-use test-strip. The test-strip holder unit according to the present invention comprises electrical leads which allow to separately connect each of the electrical leads of a test-strip or a test-strip array with a digital readout-meter device. The test-strip holder unit further comprises an interface part for receiving a test-strip or a test-strip array and an interface part to connect to the receiving module of the readout-meter. By using a release mechanism, a test-strip or a test-strip array can be released from the test-strip holder unit. The test-strip holder unit is detachable, for example by plugging/unplugging, from the readout-meter device, if necessary, e.g. for cleaning. The test-strip holder unit may have any size and shape such as a rectangular shape, a rod-like shape, or a tube-like shape. The test-strip holder unit has a main body which may be rigid or flexible, wherein preferably, for the commodity of use for the user, said main body is a rigid main body. Said test-strip holder unit comprises at least one interface suitable for connecting to an interface of a single-use test-strip or a single-use test-strip array, and comprises one interface suitable for connecting to a receiving module of a readout-meter device. In one embodiment, said test-strip holder unit may take the form of a socket or have a rotable port for connecting to a readout-meter device.

The term "potentiometric", as used herein, relates to the measurement of differences in electric potential of electrodes, e.g. the potential difference between a reference electrode and an analyte-selective working electrode, under the conditions of zero (or negligible) current. A potentiometric measurement does not involve measurement of electric current, and does not involve use of enzymes and measuring products of enzymatic reactions, nor does the quantitative determination involve any chemical such as oxidation/reduction reaction or any hydrolysis of an analyte. A measurement achieved with a POC device of the present invention and a test-strip according to the present invention is a potentiometric measurement. A potentiometric measurement according to the present invention is not based on a colorimetric or amperometric measurement. Typically, the potentiometric signal or measurement that can be taken from a test-strip according to the present invention is an electromotive force which can then be related/converted into an analyte concentration.

The term "potentiometric measurement", as used herein, in one embodiment, relates to the measurement of an analyte concentration by means of differences in potential of an analyte-selective working electrode and a reference electrode of a test-strip, wherein a reference electrode has a constant potential and an analyte-selective working electrode has a potential that develops following Nernst-linearity in relation to the amount of said analyte in a sample. In one embodiment, such a measurement is performed by contacting a single-use potentiometric test-strip, which is or was in contact with a urine sample, directly or via a test-strip holder unit to a digital readout-meter device of the present invention's POC device, thereby measuring the potential difference between the two electrodes, amplifying the signal, and converting the generated analog signal to a digital signal.

The term "depletion", as used herein, relates to a disorder of a human body or an animal body, which is characterized by a pathological decrease, i.e. deficiency, of a certain analyte in the body. The deficiency may derive from low intake of such analyte or increased loss of such analyte.

The term "overload", as used herein, relates to a disorder of a human body or an animal body, which is characterized by a pathological increase, i.e. overload, of a certain analyte in the body. The overload may derive from high intake of such analyte or decreased loss of such analyte.

The term "physiologically adequate value" or "physiologically adequate value for the patient", as used herein, relates to a value of an analyte concentration or to a value of analyte concentration ratios which is within the range of values that may be measured in humans or animals that do not have a depletion or overload of said analyte. In one embodiment, such a physiologically adequate value may depend on the constitution of the human or animal. In one embodiment, such a physiologically adequate value may depend on the age, sex, health condition, or nutritional status of a human or animal.

**BRIEF DESCRIPTION OF THE FIGURES**

The present invention is now further described by reference to the following figures, wherein

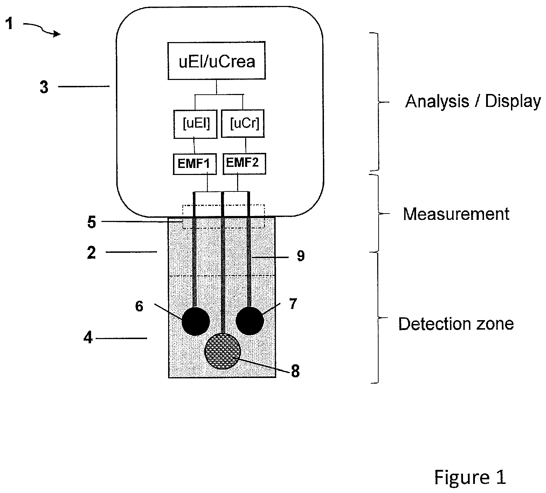

**Figure** 1 shows a schematic representation of an embodiment of a point-of-care (POC) device for detecting depletion and/or overload of an electrolyte in a patient's body; EMF = electromotive force; uEl = concentration of a cationic electrolyte in a urine sample (El= $K^+$, $Ca^{2+}$, $Mg^{2+}$, $Zn^{2+}$, $Cu^{2+}$); uCrea = creatinine concentration in a urine sample; uEl/uCrea = ratio cationic electrolyte : creatinine concentration. 1 = POC device, 2 = test-strip, 3 = readout-meter device, 4 = electrode assembly, 5 = interface for electrical connection, 6 = cationic electrolyte-selective electrode, 7 = creatinine-selective electrode, 8 = reference electrode, 9 = electrical leads

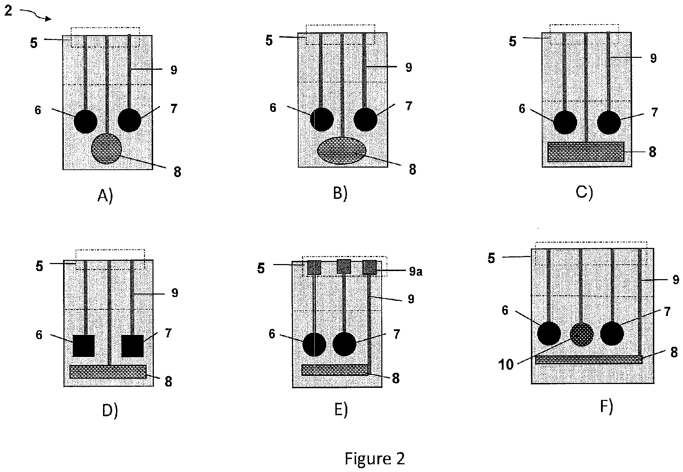

**Figure 2** shows top views of exemplary test strips with exemplary possible patterns of an electrode array and electrical leads applied on an insulating layer

   A) exemplary round shape of working electrodes + round shape of reference electrode

         5 = interface for electrical connection
         6 = cationic electrolyte-selective electrode
         7 = creatinine-selective electrode
         8 = reference electrode
         9 = electrical leads

   B) exemplary round shape of working electrodes + oval reference electrode
   C) exemplary round shape of working electrodes + rectangular reference electrode
   D) exemplary square shape of working electrodes + rectangular reference electrode
   E) 9a) = exemplary contact paths at the end of electrical leads for contacting readout-meter device
   F) 10 = neutral electrode for determining interferences

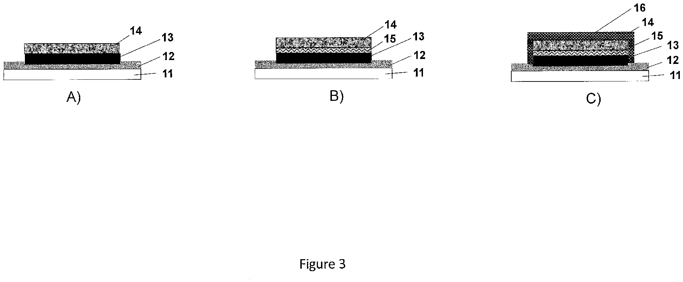

**Figure 3** shows cross-sectional views of an exemplary analyte-selective electrode

   A) Without "inner contact layer"

         11 = substrate
         12 = insulating layer
         13 = conductive layer
         14 = analyte-selective membrane

   B) With "Inner contact layer"

         15 = optionally, "inner contact layer" (transducer)

   C) With "covering layer" and "

         16= optionally, "covering layer"

**Figure 4** shows an exemplary embodiment for the fabrication of an exemplary test-strip with an additional covering layer. In such exemplary fabrication method, the following steps are performed:

   Step 1) Provide substrate with insulating layer on top
   Step 2) Apply electrode assembly and electrical leads
   Step 3) Form analyte-selective electrodes
   Step 4) preferably, apply a suitable covering film, e.g. a plastic insulating material with openings for the electrodes

   2a = test-strip with covering layer
   4a = electrode assembly and electrical leads

5 = interface for electrical connection
11a = substrate with insulating layer
15a = analyte-selective membrane solutions
16 = covering layer with openings for electrodes

**Figure 5** shows the potentiometric response of fabricated test-strips (K1-K4) according to the present invention to different concentrations of potassium ($K^+$) in aqueous solutions.

**Figure 6** shows a potentiometric response of exemplary fabricated test-strips in accordance with the present invention (Cal - Ca4) to different calcium ($Ca^{2+}$) concentrations in aqueous solutions.

**Figure 7** shows a potentiometric response of exemplary fabricated test-strips in accordance with the present invention (Creal - Crea4) to different protonated creatinine concentrations in aqueous solutions.

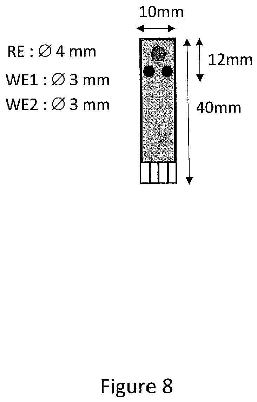

**Figure 8** shows top view of an exemplary test strip with exemplary dimensions of the electrode array and substrate. WE1 = a first working electrode; WE2 = a second working electrode; RE = reference electrode.

**Figure 9** shows an exemplary embodiment of a point-of-care (POC) device for detecting electrolyte depletion and/or electrolyte overload in a patient's body consisting of

A) a test-strip and readout-meter having a release button (17a)
B) a test-strip and readout-meter equipped with a test-strip holder (18) and a release button (17a)
C) a test-strip and readout-meter equipped with a test-strip holder (18) having a release button in form of a lever (17b)

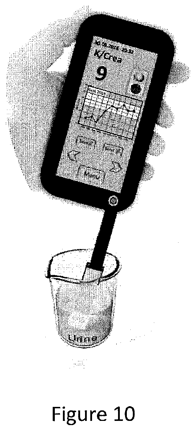

**Figure 10** shows an exemplary point-of-care (POC) device for detecting electrolyte depletion and/or electrolyte overload in a patient's body consisting of a test-strip and readout-meter displaying exemplary analysis results.

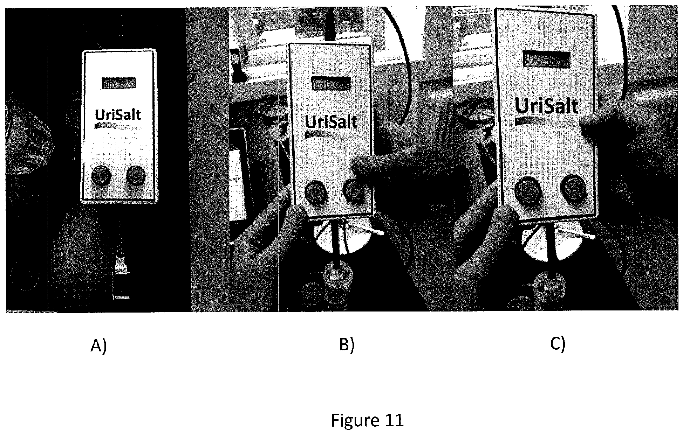

**Figure 11** shows a prototype of a (POC) device in accordance with embodiments of the present invention.

Panel A shows a (POC) device comprising a single-use test-strip connected to a readout-meter device via a test-strip holder unit, prepared for measurement;
Panel B shows a (POC) device, where the test-strip is contacting the urine sample and after the measurement mechanism had been activated to initiate a measurement in a "time-controlled manner";
Panel C shows a POC device after the measurement, where the result just after the measurement is displayed via the output unit, i.e. display, of the readout-meter to which a single-use test-strip is connected via a test-strip holder unit.

Furthermore, the present invention is now further described by reference to the following examples which are given to illustrate, not to limit the present invention.

## EXAMPLES

### Example 1

#### Structure of a test-strip

For proof of principle experiments, test-strips with electrode array exhibiting dimensions according to the present invention were fabricated. The system consists of a carbon working electrode (WE) with $\varnothing$ = 3 mm, a (Ag/AgCl) counter/reference electrode (RE), electrical leads that were screen printed onto a plastic substrate and interface for electrical connection to a readout meter.

### Example 2

#### Fabrication of a potassium-selective test-strip

To realize a potassium-selective electrode (K-ISE), a solution of poly(3-octylthiophene) in tetrahydrofuran (0.5 mg/1mL) was casted (2.5 $\mu$L) on the area of the carbon WE of test-strip of Example 1. Then the substrate was dried to remove solvent forming a thin transducer layer. Subsequently, a solution of a potassium-selective membrane (K-ISM) solution (3,5 $\mu$L) was casted on the top of that transducer layer. Then, the substrate was dried to remove solvent forming a potassium-selective membrane on the WE. The K-ISM solution consisted of a mixture of 2.0 mg potassium ionophore valinomycin, 0.5 mg potassium tetrakis(4-chlorophenyl) borate, 32.8 mg PVC (high molecular weight polyvinylchloride) and 64.7 mg bis(2-ethylhexyl) sebacate in 1 mL tetrahydrofuran.

**Example 3**

**Potentiometric measurements with the potassium-selective test-strip**

The potassium-selective test-strip was dipped into the sample solution until all electrodes were covered and the potential difference (EMF) between modified WE(s) and RE was measured with a high input impedance voltmeter that was integrated in a prototype of a readout meter having receiving module for receiving the interface of the test-strip and a display, where result was immediately displayed.

**Example 4**

**Sensor calibration by measuring a standard potassium solution series**

Standard solutions with potassium concentrations of 1M, $10^{-1}$M, $10^{-2}$M, $10^{-3}$M respectively, were prepared by dissolving potassium chloride (KC1) in water. The EMF values were recorded and a calibration curve was set up by plotting the EMF values as a function of the minus logarithm of potassium concentrations. Four different test strips (K1 - K4) were fabricated and tested. The results are summarized in Table 1.

**Table** 1. EMF values for four different potassium concentrations (1.0 - 0.001 M) obtained by measurements with four different test strips (K1 - K4).

| Concentration | | EMF | | | |
|---|---|---|---|---|---|
| | | **K1** | **K2** | **K3** | **K4** |
| KCl [mM] | -log[$K^+$] | [mV] | [mV] | [mV] | [mV] |
| 1,0 | 3 | -116 | -111 | -109 | -107 |
| 10,0 | 2 | -5 | -3 | 0 | 3 |
| 100,0 | 1 | 100 | 100 | 105 | 106 |
| 1000,0 | 0 | 200 | 203 | 208 | 209 |

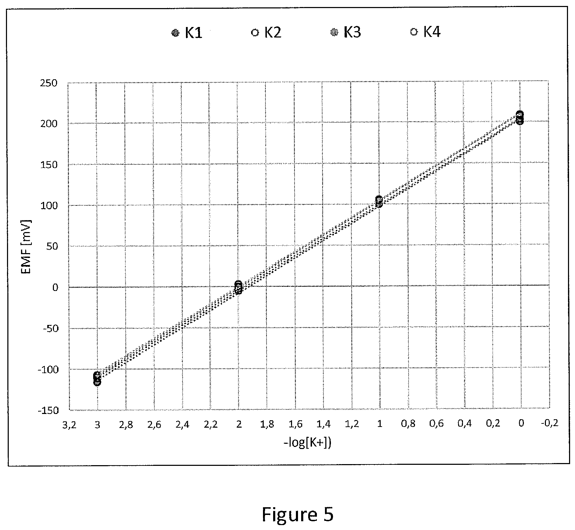

The data clearly show good reproducibility and - as shown in Fig. 5 - a linear (dynamic) range between 0.001M and 1M potassium solution. Thus, the linear range of the test strips covers medically relevant concentrations in physiological fluids as in human urine, where the range for potassium concentrations typically lies between 0.025-0.125M.
Out of these data for each test strip a regression equation of type

$$EMF = slope \; x \; (-log[K^+]) + intercept,$$

their average and deviation values were calculated (Table 2).

**Table 2.** Overview of linear regression equations and correlation coefficients obtained for EMF measurements with four different sodium sensors (K1 - K4); slope in [mV/(-log[$K^+$])], intercept in [mV], $R^2$ = coefficient of determination.

| Test-strip | slope | intercept | $R^2$ |
|---|---|---|---|
| K1 | -105,3 | 202,7 | 0,9995 |
| K2 | -104,5 | 204,0 | 0,9999 |
| K3 | -105,6 | 208,0 | 0,9998 |
| K4 | -105,1 | 210,4 | 0,9997 |

(continued)

| Test-strip | slope | intercept | $R^2$ |
|---|---|---|---|
| **average** | **-105,1** | **206,3** | **0,9997** |
| deviation | 0,4 | 3,1 | 0,0002 |

Using these regression equations, the concentration of potassium in a sample can be determined from the measured EMF as illustrated by following example:

EMF of a sample measured:     2.0 mV
Regression equation (averaged) :

$$EMF = -105.1\,(-\log[K]) + 206.3$$

than

$$-\log[K^+] = (2-206.3)\,/\,(-105.1) = \; 1{,}9439$$

$$[K^+] = 10^{-1.9439} = 0.0114\,M = \; 11.4\,mM$$

**Example 5**

**Fabrication of a calcium-selective test-strip**

To realize a calcium-selective electrode (Ca-ISE), a solution of poly(3-octylthiophene) in tetrahydrofuran (0.5 mg/1mL) was casted (2.5 μL) on the area of the carbon WE. Then the substrate was dried to remove solvent forming a thin transducer layer. Subsequently, a solution of a calcium-selective membrane (Ca-ISM) solution (3,5 μL) was casted on the top of that transducer layer. Then, the substrate was dried to remove solvent and to form a calcium-selective membrane on the WE. The Ca-ISM solution consisted of a mixture of 2.0 mg calcium ionophore II (N,N,N',N'-tetra[cyclohexyl]diglycolic acid diamide), 0.5 mg potassium tetrakis(4-chlorophenyl) borate, 32.8 mg PVC (high molecular weight polyvinylchloride) and 64.7 mg 2-Nitrophenyl octyl ether in 1 mL tetrahydrofuran.

**Example 6**

**Potentiometric measurements with the calcium-selective test-strip**

The calcium-selective test-strip was dipped into the sample solution until all electrodes were covered and the potential difference (EMF) between modified WE(s) and RE was measured with a high input impedance voltmeter that was integrated in a prototype of a readout meter having receiving module for receiving the interface of the test-strip and a display, where result was immediately displayed.

**Example 7**

**Sensor calibration by measuring a standard calcium solution series**

Standard solutions with calcium concentrations of $10^{-1}$M, $5\times10^{-2}$M, $10^{-2}$M, $5\times10^{-3}$M respectively, were prepared by dissolving calcium chloride ($CaCl_2$) in water. The EMF values were recorded and a calibration curve was set up by plotting the EMF values as a function of the minus logarithm of calcium concentrations. Four different test strips (Ca1 - Ca4) were fabricated and tested. The results are summarized in Table 3.

**Table 3.** EMF values for four different calcium concentrations (0.1 - 0.005 M) obtained by measurements with four different test strips (Cal - Ca4).

| Concentration | | EMF | | | |
|---|---|---|---|---|---|
| | | Ca1 | Ca2 | Ca3 | Ca4 |
| $CaCl_2$ [mM] | -log(c) | [mV] | [mV] | [mV] | [mV] |
| 5,0 | 2,3 | 159 | 157 | 158 | 155 |
| 10,0 | 2 | 171 | 168 | 170 | 167 |
| 50,0 | 1,3 | 211 | 212 | 212 | 210 |
| 100,0 | 0 | 229 | 228 | 228 | 224 |

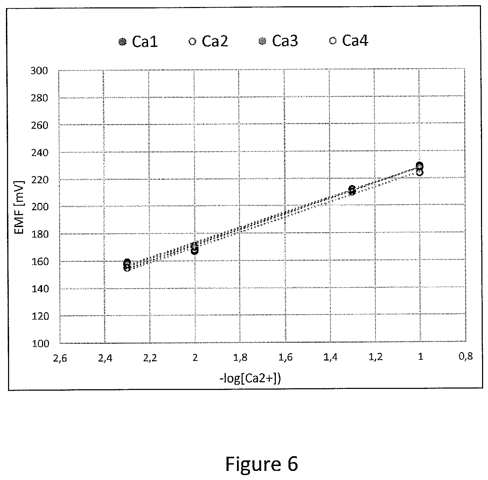

The data clearly show good reproducibility and - as shown in Fig. 6 - a linear (dynamic) range between 0.005 M and 0.1 M calcium solution. Thus, the linear range of the test strips covers medically relevant concentrations in physiological fluids as in human urine, where the calcium concentrations typically lies around 10 mM range.

Out of these data for each test strip a regression equation of type

$$EMF = slope \times (-\log[Ca^{2+}]) + intercept,$$

their average and deviation values were calculated (Table 4).

| Test-strip | slope | intercept | $R^2$ |
|---|---|---|---|
| Ca1 | -54,6 | 282,6 | 0,9966 |
| Ca2 | -56,5 | 284,4 | 0,9945 |
| Ca3 | -55,2 | 283,1 | 0,9966 |
| Ca4 | -55,0 | 279,7 | 0,9957 |

| average | -55,3 | 282,4 | 0,9959 |
|---|---|---|---|
| deviation | 0,7 | 1,7 | 0,0009 |

**Table 4.** Overview of linear regression equations and correlation coefficients obtained for EMF measurements with four different sodium sensors (Ca1 – Ca4); slope in $[mV/(-\log[Ca^{2+}])]$, intercept in [mV], $R^2$ = coefficient of determination.

Using these regression equations, the concentration of potassium in a sample can be determined from the measured EMF as illustrated by following example:

EMF of a sample measured:     155.0 mV

Regressionequation (averaged) :

$$EMF = -55.3 \, (-\log[Ca^{2+}]) + 282.4$$

than

$$-\log [Ca^{2+}] = (155-282.4) / (-55.3) = 2,3038$$

$$[Ca^+] = 10^{-2,3038} = 0.00498 \, M = 4.98 \, mM$$

**Example 8**

**Fabrication of a creatinine-selective test-strip**

To realize a creatinine-selective electrode (Crea-ISE), a solution of poly(3-octylthiophene) in tetrahydrofuran (0.5 mg/1mL) was casted (2.5 $\mu$L) on the area of the carbon WE. Then the substrate was dried to remove solvent forming a thin transducer layer on the surface. Subsequently, a solution of a creatinine-selective membrane (Crea-ISM) solution (3,5 $\mu$L) was casted on the top of that transducer layer. Then, the substrate was dried to remove solvent and to form a creatinine-selective membrane on the WE. The Crea-ISM solution consisted of a mixture of 4.0 mg creatinine molybdophosphate, 63 mg PVC (high molecular weight polyvinylchloride) and 125 mg o-Nitrophenyl octyl ether in a mixture of 2 mL tetrahydrofuran and 0.5 mL acetone.

**Example 9**

**Potentiometric measurements with the creatinine-selective test-strip**

The creatinine-selective test-strip was dipped into the sample solution until all electrodes were covered and the potential difference (EMF) between modified WE(s) and RE was measured with a high input impedance voltmeter that was integrated in a prototype of a readout meter having receiving module for receiving the interface of the test-strip and a display, where result was immediately displayed.

**Example 10**

**Sensor calibration by measuring a standard creatinine solution series**

Standard solutions with creatinine concentrations of 1M, 316.2x10$^{-1}$M, 17.8x10$^{-1}$M, 10$^{-1}$M, 3.16x10$^{-2}$M, 10$^{-2}$M, 10$^{-3}$M respectively, were prepared by dissolving creatinine hydrochloride in water. The EMF values were recorded and a calibration curve was set up by plotting the EMF values as a function of the minus logarithm of potassium concentrations. Four different test strips (Crea1 - Crea2) were fabricated and tested. The results are summarized in Table 5.

Table 5. EMF values for seven different creatinine concentrations (1.0 - 0.001 M) obtained by measurements with four different test strips (Crea1 - Crea4).

| Concentration | | EMF | | | |
|---|---|---|---|---|---|
| | | Crea1 | Crea2 | Crea3 | Crea4 |
| [mM] | log(Crea) | [mV] | [mV] | [mV] | [mV] |
| 1,0 | 3 | 34 | 23 | 22 | 33 |
| 10,0 | 2 | 147 | 137 | 136 | 147 |
| 31,6 | 1,5 | 204 | 193 | 191 | 204 |
| 100,0 | 1 | 259 | 247 | 245 | 258 |
| 177,8 | 0,75 | 284 | 274 | 270 | 281 |
| 316,2 | 0,5 | 312 | 302 | 298 | 311 |
| 1000,0 | 0 | 362 | 354 | 349 | 357 |

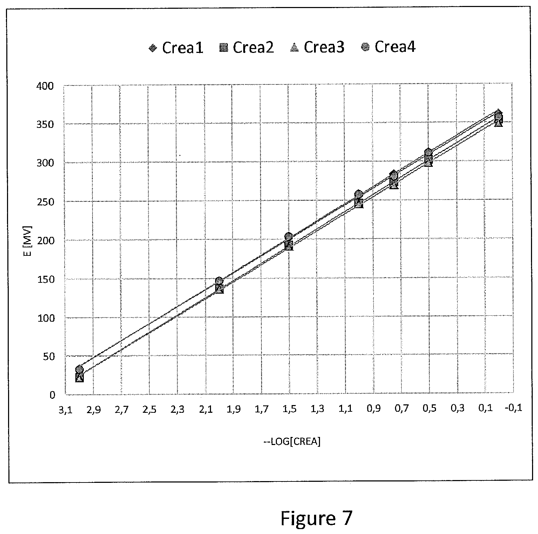

The data clearly show good reproducibility and - as shown in Fig. 7- a linear (dynamic) range between 0.001M and 1M creatinine solution. Thus, the linear range of the test strips covers medically relevant concentrations in physiological fluids as in human urine, where the range for creatinine concentrations typically lies around 1-32 mM.

Out of these data for each test strip a regression equation of type

$$EMF = slope \times (-\log[Crea]) + intercept,$$

their average and deviation values were calculated (Table 6).

| Test-strip | slope | intercept | $R^2$ |
|---|---|---|---|
| Crea1 | -109,8 | 366,1 | 0,9995 |
| Crea2 | -110,4 | 356,6 | 0,9997 |

| Crea3 | -109,0 | 352,1 | 0,9996 |
|---|---|---|---|
| Crea4 | -108,7 | 363,1 | 0,9988 |
| average | -109,8 | 358,3 | 0,9996 |
| deviation | 0,6 | 5,8 | 0,0001 |

Using these regression equations, the concentration of potassium in a sample can be determined from the measured EMF as illustrated by following example:

EMF of a sample measured: 140.0 mV
Regression equation (averaged): EMF=-109.8(-log[Crea]) + 358,3
than

$$-\log [\mathrm{Crea}] = (140.0 - 358.3) / (-109.8) = 1,9882$$

$$[\mathrm{Crea}] = 10^{-1.9882} = 0.01027 \,\mathrm{M} = \qquad 10.3 \,\mathrm{mM}$$

## REFERENCES

[1] Fluids, Electrolytes and Acid-Base Disorders Handbook, Editors: Canada TW, Tajchman SK, Tucker AM, Ybarra JV. ASPEN (2015); eBook edition: ISBN-13: 978-1-889622-18-7

[2] McDonough AA, Youn JH. Potassium homeostasis: the knowns, the unknowns and the health benefits. Physiology 32:100-11(2017)

[3] Unwin RJ, Luft FC, Shirley DG. Pathophysiology and management of hypokalemia: a clinical perspective. Nat Rev Nephrol 7:75-84(2011)

[4] Glassock RJ, Goldstein DA, Goldstone R, HsuehWA. Diabetes mellitus, moderate renal insufficiency and hyperkalemia. Am J Nephrol 3:233-40(1983)

[5] Barstow C. Calcium disorders. FP Essent 459:29-34(2017)

[6] Gallagher JC, Smith LM, Yalamanchili V. Incidence of hypercalciuria and hypercalcemia during vitamin D and calcium supplementation in older women. Menopause 21:1173-80(2014)

[7] Taufield PA, Ales KL, Resnick LM, Drusin ML, Gertner JM, Laragh JH. Hypocalciuria in preeclampsia.N Engl J Med 316:715-8(1987)

[8] Volpe SL. Magnesium in disease prevention and overall health. Adv Nutr 4:378S-83S(2013)

[9] Menditto VG, Lucci M, Polonara S. The role of hypomagnesuria in urolithiasis and renal colic: results from a prospective study of a metabolic protocol. Minaerva Med 103:377-82(2012)

[10] Gordon A, Magee LA, Payne B, Firoz T, Sawtchuk D, Tu D, Vidler M, de Silva D, Dadelszen P. Magnesium sulphate for the management of preeclampsia and eclampsia in middle and low-income countries: a systematic review of dosing regimens. J Obstet Gynecol Can 36:154-63(2014)

[11] Ackland ML, Michalczyk A. Zinc deficiency and its inherited disorders -a review. Genes Nutr 1(1); 41-50(2006)

[12] Romana DL, Olivares M, Uauy R, Araya M. Risks and benefits of copper in light of new insights of copper homeostasis. J Trace Elem Med Biol 25; 3-13(2011)

[13] Vashist SK, Luppa PB, Yeo L Y, Ozcan A, Luong JHT, Emerging Technologies for Next-Generation Point-of-Care Testing. Trends in Biotechnology, 33, 11; 692-705(2015)

[14] Gubala et al. Point of Care Diagnostics: Status and Future. Anal. Chem. 84, 487-515(2012)

[15] Koo H, Lee SG, Kim JH. Evaluation of random urine sodium and potassium compensated by creatinine as possible alternative for 24-hour urinary sodium and potassium excretion. Ann Lab Med 35:238-241(2015)

[16] Tang NLS, Chan YK, Hui E., Woo J. Application of Urine Magnesium/Creatinine Ratio as an Indicator for Insufficient Magnesium Intake. Clin. Biochemi. 33, 8, 675-678 (2000)

[17] Newman DJ, Price CP. Renal function and nitrogen metabolites. In Tietz Textbook of Clinical Chemistry. Third Edition. Edited by Burtis CA, Ashwood ER. pp 1204-1270(1990)

[18] Newman JD, Turner APF, Home blood glucose biosensors: a commercial perspective. Biosensors and Bioelectronics 20; 2435-2453(2005)

[19] Thomas L. Labor und Diagnose. TH Books (2012)

[20] Syal K., Banerjee D., Srinivasan A. Creatinine estimation and interference. Ind J Clin Biochem 28(2); 210-211(2013)

[21] C.S. Pundir CS. Sandeep Yadav S. Kumar A. Creatinine sensors, TRAC 50; 2-52 (2013)

[22] http://www.novabio.us/uk/statstrip-creatinine/

[23] Shephard MDS, Point-of-Care Testing and Creatinine Measurement, Clin Biochem Rev, 32; 109-114 (2011)

The features of the present invention disclosed in the specification, the claims, and/or in the accompanying figures may, both separately and in any combination thereof, be material for realizing the invention in various forms thereof.

## Claims

1. A single-use test-strip for the quantitative determination of a concentration of a cationic electrolyte which is not sodium, and of creatinine concentration in a patient's urine sample,
said test-strip comprising:

   - a substrate which either is electrically insulating or which has an electrically insulating layer applied thereon,
   - an electrode assembly applied on said substrate or on said electrically insulating layer, if present,
   said electrode assembly comprising at least

      - one working electrode that is selective for said cationic electrolyte;
      - one creatinine-selective working electrode;
      - either one joint reference electrode for both said cationic electrolyte-selective working electrode and said creatinine-selective working electrode, or a reference electrode for said cationic electrolyte-selective working electrode and a separate reference electrode for said creatinine-selective working electrode;
      - optionally; one or two neutral electrodes for measuring and eliminating interferences,

   - an interface for electrically connecting said electrode assembly to a read out-meter device.

2. The single-use test-strip according to claim 1, wherein said working electrodes, said reference electrode(s) and said neutral electrode(s), if present, have been applied on said substrate or on said electrically insulating layer, if present,

by a suitable deposition technique, such as printing, sputtering, evaporating, electro-less plating, affixation, gluing or lithography, preferably screen printing or ink-jet printing, thus forming an electrode assembly on said substrate or on said electrically insulating layer, and wherein said cationic electrolyte-selective working electrode comprises a cationic electrolyte-selective membrane, and said creatinine-selective working electrode comprises a creatinine-selective membrane, and wherein said neutral electrode(s) comprises(comprise) a membrane that is not selective for said cationic electrolyte and not creatinine-selective.

3. The single-use test-strip according to any of the foregoing claims, wherein said cationic electrolyte which is not sodium is selected from the group comprising, preferably consisting of, potassium, calcium, magnesium, zinc and copper.

4. The single-use test strip according to any of the foregoing claims, wherein said substrate is made of a material selected from plastic, ceramic, alumina, paper, cardboard, rubber, textile, carbon-based polymers, such as polypropylene, fluoropolymers, such as Teflon, silicon-based substrates, such as glass, quartz, silicon nitride, silicon oxide, silicon based polymers such as polydimethoxysiloxane, semiconducting materials such as elemental silicon, dielectric materials, preferably selected from organic dielectric materials such as polyimide, polycarbonate, polyvinyl chloride, polystyrene, polyethylene, polypropylene, polyester, polyethylene terephthalate, polyurethane, polyvinylidene fluoride, inorganic dielectric materials such as silicium dioxide, and wherein said electrically insulating layer, if present, is made of a dielectric material, wherein, if said electrically insulating layer is present on said substrate, said electrode assembly is located on said electrically insulating layer.

5. The single-use test-strip according to any of claims 2-4, wherein said cationic electrolyte-selective working electrode comprises a cationic electrolyte-selective membrane that comprises a cationic electrolyte-selective carrier in a polymer matrix, and said creatinine-selective membrane comprises a creatinine-selective carrier in a polymer matrix.

6. The single-use test-strip according to any of the foregoing claims, wherein said electrode assembly further comprises one or two neutral electrodes for measuring and eliminating interferences, wherein said neutral electrode(s) comprise a membrane comprising a polymeric matrix without any cationic electrolyte-selective carrier and without any creatinine-selective carrier.

7. The single-use test-strip according to any of the foregoing claims, wherein each of said electrodes in said electrode assembly has an electrical lead, respectively, wherein said electrical lead connects said electrode with said interface for electrically connecting said electrode assembly to a readout-meter device.

8. The single-use test-strip according to any of the foregoing claims, wherein said joint reference electrode has a surface larger than the surface of each of said working electrodes, or each of said separate reference electrodes has a surface larger than the surface of each of said working electrodes.

9. A non-invasive point-of-care (POC) device for detecting a disorder of electrolyte balance in a patient's body, said POC device comprising:

- a readout-meter-device for the quantitative and selective measurement of cationic electrolyte concentration and creatinine concentration in a urine sample and for determining a ratio of cationic electrolyte-to-creatinine, said readout-meter-device comprising
- a receiving module for receiving an interface of a single-use test-strip according to any of claims 1-8 and for establishing electrical contact between said readout-meter-device and an electrode assembly of said single-use test-strip, thus allowing the detection and transmission of electrical signal(s) from said single-use test-strip to said readout-meter-device, wherein said receiving module has electrical connectors for separately contacting each electrode via said interface of said test-strip
- a multichannel amplifier, preferably having high input resistance, for amplifying electrical signal(s) transmitted from a single-use test-strip according to any of claims 1-8
- a controller including an analog/digital converter and a storage memory, for converting electrical signals received from a single-use test-strip according to any of claims 1 - 8 into cationic electrolyte concentration measurement(s) and creatinine concentration measurement(s) and for subsequently determining a ratio of cationic electrolyte concentration to creatinine concentration based on said cationic electrolyte concentration measurements and creatinine concentration measurements
- an output device for indicating concentration measurements and/or said ratio to a user, preferably a display, and
- a power supply.

**10.** The non-invasive point-of-care (POC) device according to claim 9, further comprising

    - a single-use test-strip according to any of claims 1-8 inserted into said receiving module of said readout-meter-device by way of said interface of said single-use test-strip, thus establishing electrical contact between said electrode assembly of said test-strip and said readout-meter device.

**11.** The non-invasive point-of-care (POC) device according to any of claims 9-10, wherein said device further comprises

    - a user-interface for operating said device, and/or a memory for storing a plurality of cationic electrolyte and creatinine concentration measurements and determined ratios of cationic electrolyte concentration to creatinine concentration, and/or a connection interface, preferably a USB and/or wireless connection interface, for transferring and/or exchanging data with an external computer or external network.

**12.** A method for quantitatively determining cationic electrolyte concentration and creatinine concentration in a patient's urine sample, comprising the steps:

    a) providing a patient's urine sample,
    b) contacting a single-use test-strip according to any of claims 1-8 with said urine sample and allowing the electrode assembly of said test-strip to be wetted by and come into contact with said urine sample, optionally withdrawing the urine-wetted test-strip from said urine sample,
    c) connecting said test-strip to a readout-meter-device of a point-of-care (POC) device as defined in claim 9, to assemble a point-of-care (POC) device as defined in claim 10, wherein said single-use test-strip is inserted into said receiving module of said readout-meter device, thus establishing electrical contact between said electrode assembly of said test-strip and said readout-meter-device,
    wherein said connecting of said test strip to said readout-meter device of said point of care in step c) occurs either before or after step b),
    d) measuring cationic electrolyte concentration and creatinine concentration in said urine sample, using said point-of-care (POC) device assembled in step c).

**13.** A method of detecting a disorder of electrolyte balance in a patient's body, said method comprising the steps:

    - Performing the method according to claim 12,
    - determining a ratio of cationic electrolyte concentration to creatinine concentration using said point-of-care (POC) device,
    - detecting a disorder of electrolyte balance, if said ratio of cationic electrolyte concentration to creatinine concentration in said urine sample, as determined in the previous step, is outside of, i.e. higher or lower than, a range of physiologically adequate ratio values for the respective patient.

**14.** The method according to claim 13, wherein said disorder of electrolyte balance is a cationic electrolyte depletion or cationic electrolyte overload, and is, preferably, selected from:

    - a potassium depletion in the plasma of a patient e.g. due to increased renal and/or gastrointestinal loss of potassium, such as may occur in acquired or genetic kidney diseases, during diuretics intake or prolonged vomiting and/or diarrhea during infections, inflammatory, malignant and genetic bowel diseases,
    - a potassium overload in the plasma of a patient e.g. due to intake of drugs raising potassium level, or during acute kidney injury, cardiovascular disease and diabetes mellitus,
    - a calcium depletion in the body of a patient e.g. due to metabolic disorders, Vitamin D deficiency or enhanced renal calcium loss, such as may occur in kidney stones disease, osteoporosis, and diabetes mellitus,
    - a calcium overload in the body of a patient e.g. due to decreased renal calcium loss, such as may occur in pre-eclampsia during pregnancy,
    - a magnesium depletion or magnesium overload in the body of a patient, such as may occur in urolithiasis, migraine, Alzheimer's disease, coronary heart disease, hypertension, diabetes mellitus type 2, and pre-eclampsia and eclampsia disease,
    - a zinc depletion in the body of a patient e.g. due to dietary inadequacy or gastrointestinal disorders, such as may occur in celiac or Crohn's disease, Wilson's disease, diabetes, chronic liver and kidney disease,
    - a copper depletion in the body of a patient e.g. due to malabsorption and malabsorption, such as may occur during nephrotic syndrome, Menkes, disease and hypoproteinemias, and
    - a copper overload in the body of a patient, due to hampered renal excretion of copper, such as may occur in Wilson's disease.

15. The method according to any of claims 13-14, wherein said range of physiologically adequate ratio values is or has been separately determined by reference to a healthy person, preferably of the same age, sex and/or body weight.

Figure 1

Figure 2

A)

B)

C)

Figure 3

EP 3 640 633 A1

Figure 4

30

Figure 5

Figure 6

Figure 7

RE : Ø 4 mm

WE1 : Ø 3 mm

WE2 : Ø 3 mm

Figure 8

Figure 9

Figure 10

A)                              B)                              C)

Figure 11

Europäisches
Patentamt

European
Patent Office

Office européen
des brevets

## EUROPEAN SEARCH REPORT

Application Number

EP 18 20 0692

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2016 0127310 A (UNIV KEIMYUNG IND ACAD COOP FOUND [KR]) 3 November 2016 (2016-11-03) * abstract * * page 7, legend of figure 4.; figure 5 * ----- | 1-15 | INV. G01N27/327 |
| A | FANG WEI ET AL: "Serum Creatinine Detection by a Conducting-Polymer-Based Electrochemical Sensor To Identify Allograft Dysfunction", ANALYTICAL CHEMISTRY, vol. 84, no. 18, 18 September 2012 (2012-09-18), pages 7933-7937, XP055241019, US ISSN: 0003-2700, DOI: 10.1021/ac3016888 * the whole document * ----- | 1-15 | |
| A | NELSON L.S TANG ET AL: "Application of Urine Magnesium/Creatinine Ratio as an Indicator for Insufficient Magnesium Intake", CLINICAL BIOCHEMISTRY, vol. 33, no. 8, 1 November 2000 (2000-11-01), pages 675-678, XP055587324, US, CA ISSN: 0009-9120, DOI: 10.1016/S0009-9120(00)00173-9 * the whole document * ----- | 1-15 | TECHNICAL FIELDS SEARCHED (IPC) G01N |
| A | WO 96/04554 A1 (N D INST AUST PTY LIMITED SA [AU] ET AL.) 15 February 1996 (1996-02-15) * the whole document * ----- -/-- | 1-15 | |

The present search report has been drawn up for all claims

| Place of search | Date of completion of the search | Examiner |
|---|---|---|
| Munich | 13 May 2019 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 18 20 0692

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LVOVA L ET AL: "Clinical analysis of human urine by means of potentiometric Electronic tongue", TALANTA, ELSEVIER, AMSTERDAM, NL, vol. 77, no. 3, 15 January 2009 (2009-01-15), pages 1097-1104, XP025760511, ISSN: 0039-9140, DOI: 10.1016/J.TALANTA.2008.08.021 [retrieved on 2008-09-02] * the whole document * ----- | 1-15 | |

| | | | TECHNICAL FIELDS SEARCHED (IPC) |

The present search report has been drawn up for all claims

| Place of search | Date of completion of the search | Examiner |
|---|---|---|
| Munich | 13 May 2019 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 18 20 0692

This annex lists the patent family members relating to the patent documents cited in  the above-mentioned European search report.
The members are as contained in the European Patent Office EDP file on
The European Patent Office is in no way liable for these particulars which are merely given for the purpose of information.

13-05-2019

| Patent document cited in search report | | Publication date | Patent family member(s) | | Publication date |
|---|---|---|---|---|---|
| KR 20160127310 | A | 03-11-2016 | NONE | | |
| WO 9604554 | A1 | 15-02-1996 | CA | 2196546 A1 | 15-02-1996 |
| | | | EP | 0775312 A1 | 28-05-1997 |
| | | | NZ | 290093 A | 23-12-1998 |
| | | | WO | 9604554 A1 | 15-02-1996 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9604554 A1


**Non-patent literature cited in the description**

- Fluids, Electrolytes and Acid-Base Disorders Handbook. ASPEN, 2015
- **MCDONOUGH AA ; YOUN JH.** Potassium homeostasis: the knowns, the unknowns and the health benefits. *Physiology,* 2017, vol. 32, 100-11
- **UNWIN RJ ; LUFT FC ; SHIRLEY DG.** Pathophysiology and management of hypokalemia: a clinical perspective. *Nat Rev Nephrol,* 2011, vol. 7, 75-84
- **GLASSOCK RJ ; GOLDSTEIN DA ; GOLDSTONE R ; HSUEHWA.** Diabetes mellitus, moderate renal insufficiency and hyperkalemia. *Am J Nephrol,* 1983, vol. 3, 233-40
- **BARSTOW C.** Calcium disorders. *FP Essent,* 2017, vol. 459, 29-34
- **GALLAGHER JC ; SMITH LM ; YALAMANCHILI V.** Incidence of hypercalciuria and hypercalcemia during vitamin D and calcium supplementation in older women. *Menopause,* 2014, vol. 21, 1173-80
- **TAUFIELD PA ; ALES KL ; RESNICK LM ; DRUSIN ML ; GERTNER JM ; LARAGH JH.** Hypocalciuria in preeclampsia. *N Engl J Med,* 1987, vol. 316, 715-8
- **VOLPE SL.** Magnesium in disease prevention and overall health. *Adv Nutr,* 2013, vol. 4, 378S-83S
- **MENDITTO VG ; LUCCI M ; POLONARA S.** The role of hypomagnesuria in urolithiasis and renal colic: results from a prospective study of a metabolic protocol. *Minaerva Med,* 2012, vol. 103, 377-82
- **GORDON A ; MAGEE LA ; PAYNE B ; FIROZ T ; SAWTCHUK D ; TU D ; VIDLER M ; DE SILVA D ; DADELSZEN P.** Magnesium sulphate for the management of preeclampsia and eclampsia in middle and low-income countries: a systematic review of dosing regimens. *J Obstet Gynecol Can,* 2014, vol. 36, 154-63
- **ACKLAND ML ; MICHALCZYK A.** Zinc deficiency and its inherited disorders -a review. *Genes Nutr,* 2006, vol. 1 (1), 41-50
- **ROMANA DL ; OLIVARES M ; UAUY R ; ARAYA M.** Risks and benefits of copper in light of new insights of copper homeostasis. *J Trace Elem Med Biol,* 2011, vol. 25, 3-13
- **VASHIST SK ; LUPPA PB ; YEO L Y ; OZCAN A ; LUONG JHT.** Emerging Technologies for Next-Generation Point-of-Care Testing. *Trends in Biotechnology,* 2015, vol. 33 (11), 692-705
- **GUBALA et al.** Point of Care Diagnostics: Status and Future. *Anal. Chem.,* 2012, vol. 84, 487-515
- **KOO H ; LEE SG ; KIM JH.** Evaluation of random urine sodium and potassium compensated by creatinine as possible alternative for 24-hour urinary sodium and potassium excretion. *Ann Lab Med,* 2015, vol. 35, 238-241
- **TANG NLS ; CHAN YK ; HUI E ; WOO J.** Application of Urine Magnesium/Creatinine Ratio as an Indicator for Insufficient Magnesium Intake. *Clin. Biochemi.,* 2000, vol. 33 (8), 675-678
- Renal function and nitrogen metabolites. **NEWMAN DJ ; PRICE CP.** Tietz Textbook of Clinical Chemistry. 1990, 1204-1270
- **NEWMAN JD ; TURNER APF.** Home blood glucose biosensors: a commercial perspective. *Biosensors and Bioelectronics,* 2005, vol. 20, 2435-2453
- Labor und Diagnose. **THOMAS L.** TH Books. 2012
- **SYAL K. ; BANERJEE D. ; SRINIVASAN A.** Creatinine estimation and interference. *Ind J Clin Biochem,* 2013, vol. 28 (2), 210-211
- **C.S. PUNDIR CS. ; SANDEEP YADAV S. ; KUMAR A.** Creatinine sensors. *TRAC,* 2013, vol. 50, 2-52
- **SHEPHARD MDS.** Point-of-Care Testing and Creatinine Measurement. *Clin Biochem Rev,* 2011, vol. 32, 109-114